(19) Europäisches Patentamt
European Patent Office
Office européen des brevets

(11) EP 3 851 518 A1

(12) **EUROPEAN PATENT APPLICATION**
published in accordance with Art. 153(4) EPC

(43) Date of publication:
**21.07.2021 Bulletin 2021/29**

(21) Application number: **19859574.6**

(22) Date of filing: **09.09.2019**

(51) Int Cl.:
***C12N 5/071*** *(2010.01)* ***C07K 14/78*** *(2006.01)*
***C12N 1/00*** *(2006.01)* ***C12N 5/074*** *(2010.01)*

(86) International application number:
**PCT/JP2019/035358**

(87) International publication number:
**WO 2020/054659 (19.03.2020 Gazette 2020/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **10.09.2018 JP 2018168957**
**25.06.2019 JP 2019117748**

(71) Applicants:
- **Tokyo Institute of Technology**
**Tokyo 152-8550 (JP)**
- **The University of Tokyo**
**Bunkyo-ku,**
**Tokyo 113-8654 (JP)**
- **Kanto Kagaku Kabushiki Kaisha**
**Tokyo 103-0023 (JP)**

(72) Inventors:
- **KUME, Shoen**
**Tokyo 152-8550 (JP)**
- **SHIRAKI, Nobuaki**
**Tokyo 152-8550 (JP)**
- **MAEDA, Kazuya**
**Tokyo 113-8654 (JP)**
- **KUSUHARA, Hiroyuki**
**Tokyo 113-8654 (JP)**
- **ISHIKAWA, Masaya**
**Isehara-shi, Kanagawa 259-1146 (JP)**
- **WATANABE, Teruhiko**
**Isehara-shi, Kanagawa 259-1146 (JP)**

(74) Representative: **J A Kemp LLP**
**14 South Square**
**Gray's Inn**
**London WC1R 5JJ (GB)**

# (54) METHOD FOR PRODUCING INTESTINAL CELLS FROM PLURIPOTENT STEM CELLS

(57) As a method for producing enterocytes derived from pluripotent stem cells and having functions corresponding to those of actual enterocytes, a method for producing enterocytes using an enterocyte differentiation medium containing a GSK3 inhibitor, and at least one selected from the group consisting of an activator of a hepatocyte growth factor receptor, an adrenal cortex hormone, calcitriol, and dimethyl sulfoxide is provided.

EP 3 851 518 A1

SEQUENCE LISTING

<110> Tokyo Institute of Technology
The University of Tokyo
KANTO CHEMICAL CO., INC.

<120> METHOD FOR PRODUCING ENTEROCYTES FROM PLURIPOTENT STEM CELLS

<130> FP-269PCT

<150> JP 2018/168957
<151> 2018-9-10

<150> JP 2019/117748
<151> 2019-6-25

<160> 18

<170> PatentIn Ver. 2.1

<210> 1
<211> 24
<212> DNA
<213> Artificial Sequence

<220>
<223> Description of Artificial Sequence:primer

<400> 1
taactggaac tgcaaacctg gaga 24

<210> 2
<211> 22
<212> DNA
<213> Artificial Sequence

<220>
<223> Description of Artificial Sequence:primer

<400> 2
ctgattgcag acggtttgag ga 22

<210> 3
<211> 20
<212> DNA
<213> Artificial Sequence

<220>
<223> Description of Artificial Sequence:primer

<400> 3
tcactgggca tttccgtgag 20

<210> 4
<211> 22
<212> DNA
<213> Artificial Sequence

<220>

<223> Description of Artificial Sequence:primer

<400> 4
gtggatcggc cagataacaa ga 22

<210> 5
<211> 24
<212> DNA
<213> Artificial Sequence

<220>
<223> Description of Artificial Sequence:primer

<400> 5
cgactgctac ctgctgctct acac 24

<210> 6
<211> 24
<212> DNA
<213> Artificial Sequence

<220>
<223> Description of Artificial Sequence:primer

<400> 6
cggcttgata agctgatgct gtaa 24

<210> 7
<211> 23
<212> DNA
<213> Artificial Sequence

<220>
<223> Description of Artificial Sequence:primer

<400> 7
ggagcctact tggtggcaca taa 23

<210> 8
<211> 24
<212> DNA
<213> Artificial Sequence

<220>
<223> Description of Artificial Sequence:primer

<400> 8
tggcatagtc aggagcaaat gaac 24

<210> 9
<211> 25
<212> DNA
<213> Artificial Sequence

<220>
<223> Description of Artificial Sequence:primer

<400> 9

gttcccaaat tgatgttgtg acaga 25

<210> 10
<211> 25
<212> DNA
<213> Artificial Sequence

<220>
<223> Description of Artificial Sequence:primer

<400> 10
gttcccaaat tgatgttgtg acaga 25

<210> 11
<211> 20
<212> DNA
<213> Artificial Sequence

<220>
<223> Description of Artificial Sequence:primer

<400> 11
tcacctgtgg cgaagtggtc 20

<210> 12
<211> 19
<212> DNA
<213> Artificial Sequence

<220>
<223> Description of Artificial Sequence:primer

<400> 12
agcagccatc ctgcctgaa 19

<210> 13
<211> 25
<212> DNA
<213> Artificial Sequence

<220>
<223> Description of Artificial Sequence:primer

<400> 13
aacactgcag ttgacttgtt tggag 25

<210> 14
<211> 22
<212> DNA
<213> Artificial Sequence

<220>
<223> Description of Artificial Sequence:primer

<400> 14
ggtttctgcc aatcacacgt tc 22

<210> 15
<211> 24
<212> DNA
<213> Artificial Sequence

<220>
<223> Description of Artificial Sequence:primer

<400> 15
aatcactgca gctgacttac ttgg 24

<210> 16
<211> 20
<212> DNA
<213> Artificial Sequence

<220>
<223> Description of Artificial Sequence:primer

<400> 16
ccggtttctg ccaatgacac 20

<210> 17
<211> 20
<212> DNA
<213> Artificial Sequence

<220>
<223> Description of Artificial Sequence:primer

<400> 17
gaaacacaga tccccctgaa 20

<210> 18
<211> 20
<212> DNA
<213> Artificial Sequence

<220>
<223> Description of Artificial Sequence:primer

<400> 18
ctggtgttct caggcacaga 20

## Description

Technical Field

The present invention relates to an enterocyte differentiation medium, a method for producing enterocytes from pluripotent stem cells, and enterocytes produced by the method.

Background Art

The intestine is an important tissue for absorption of nutrient, water and drug, and is important for drug development because absorption of a drug and metabolism determines the bioavailability of the drug. For drug development, the human colon cancer cell line Caco-2 is widely used as a model of the intestinal epithelium for testing absorptive and metabolic functions. However, Caco-2 cells derived from the large intestine show low metabolizing enzymatic activity, which does not resemble that of the small intestine (Hubatsch et al., (2007) Nat. Protoc. 2, 2111-2119; Sun et al., (2002) Pharm. Res. 19, 4-6). Moreover, the Caco-2 cells show cell line-to-cell line differences in their properties. Therefore, there is a need to establish a human *in vitro* model that resembles the human small intestine and can be used to substitute the Caco-2 cells in drug tests.

Human embryonic stem cells (hESCs) and human induced pluripotent stem cells (hiPSCs) (Takahashi et al., (2007) Cell 131, 861-72) have the potential to differentiate and give rise to all cells derived from the three germ layers, and then to specific cell types upon exposure to appropriate growth factors. Recent studies have demonstrated the differentiation of embryonic stem (ES) cells and induced pluripotent stem (iPS) cells into the definitive endoderm and its derivative organs, such as the pancreas, the liver, and the intestine.

In the intestinal epithelium, intestinal stem cells (ISCs) give rise to differentiated cells including absorptive cells and secretory cells (such as goblet cells, enteroendocrine cells, and Paneth cells) (Nakamura et al., (2007) J. Gastroenterol. 42, 705-10; Sato, T. and Clevers, H. (2013) Science 340, 190-194). Mutant mice studies have revealed a number of genes and factors involved in the maintenance and regulation of the intestinal stem cell proliferation and differentiation, such as Wnt/β-catenine and Notch signaling (Buczacki et al., (2013) Nature 495, 65-9; Chiba, (2006) Stem Cells 24, 2437-47; Fre et al., (2005) Nature 435, 964-8; Fre et al., (2009) Proc. Natl. Acad. Sci. U. S. A. 106, 6309-14; Gerbe et al., (2011) J. Cell Biol. 192, 767-80; Gregorieff et al., (2015) Nature). The ISCs express LGR5 (Barker et al., (2007) Nature 449, 1003-7). The LGR5 is a Wnt signaling receptor that mediates Wnt/β-catenine signaling upon binding of its ligand R-spondin1. Through screening for factors that maintain ISCs in organoid culture, epidermal growth factor (EGF), Noggin, and R-spondin1 have been defined as niche factors that maintain the ISCs in culture (Sato et al., (2009) Nature 459, 262-5). Since extracellular matrices are known to be important for the maintenance of ISCs, matrigel is used and growth factors are supplemented to support the proliferation of the ISCs. Later, through molecular compound screening, long-term organoid culture procedure from primary adult human small intestinal or colonic epithelial tissue (Sato et al., (2011) Gastroenterology 141, 1762-1772) has been established by supplementation of niche factors, that is, Wnt3a, EGF, Noggin, R-spondin1, nicotinamide, and A83-01 (an inhibitor for TGF-β type I receptor kinase, also known as activin-like kinase 5 (ALK5)) .

It is known that with the use of the organoid culture system for the ISCs, hiPSCs are differentiated into enterocytes by first differentiating them into definitive endoderm by activin, and then culturing the resultant in matrigel supplemented with high concentrations of FGF and Wnt (Spence et al., (2011) Nature 470, 105-9). However, these hiPSC-derived cells were with a low population of Lgr5-expressing cells and are therefore considered to be immature. It has been reported that when these iPSC-derived enterocytes were subjected to prolonged culture and then transplanted under mouse kidney capsule, these cells were further matured, and differentiated cell types were generated 6 weeks after the transplantation (Watson et al. (2014) Nat. Med. 20).

Organoid culture is a three-dimensional culture system. On the other hand, attempts for deriving intestinal epithelial cells in a two-dimensional monolayer culture have been investigated. It has been reported that FGF4 and Wnt3A differentiate the endoderm into CDX2 positive enterocytes (Ameri et al., (2010) Stem Cells 28, 45-56; Hansson et al., (2009) Dev. Biol. 330, 286-304). The group of the present inventors reported two-dimensional culture for intestinal epithelial differentiation from mouse and human embryonic stem (ES) cells. After definitive endoderm differentiation, 6-bromoindirubin-3'-oxime (BIO), that is, a glycogen synthase kinase (GSK)-3β inhibitor, and DAPT, that is, a γ-secretase inhibitor, synergistically induced CDX2-expressing posterior definitive endodermal cells. The definitive endodermal cells then differentiated into four mature intestinal cell types, namely, enterocytes, goblet cells, enteroendocrine cells, and Paneth cells (Ogaki et al., (2013) Stem Cells 31, 1086-96). Mature cells of the enterocytes were obtained through a 16 day differentiation rapid protocol (Ogaki et al., (2015) Sci. Rep. 5, 17297).

Differentiation of enterocyte-like cells from human iPS cells using several small-molecular compounds mimicking the organoid culture system has been reported, and it promoted the differentiation of human iPSCs into enterocytes. The enterocyte-like cells showed PEPT1 or BCRP expression comparable to that of the adult intestine (Iwao et al.,

(2015) Drug Metab. Dispos. 43, 603-610; Kodama et al., (2016) Drug Metab. Dispos. 44). In the hiPSC-derived enterocytes, the PEPT1 or BCRP-mediated active transport activity was evaluated by uptake of the corresponding substrate, glycylsarcosine or Hoechst 33342, and the specificity was confirmed by using the corresponding specific inhibitor, Ibuprofen or Ko-143, respectively. Recently, it was revealed that hiPSC-derived enterocytes generated by exposure to BIO and DAPT express several major transporters, CYP3A4 and its transcription regulators (Non Patent Literatures 1 and 2).

Citation List

Non Patent Literature

Non Patent Literature 1: Ozawa, T. et al., (2015) Sci. Rep., 5:16479
Non Patent Literature 2: Negoro, R. et al., (2016) Res. Commun. 472, 631-6

Summary of Invention

Technical Problem

For pharmacokinetic studies, an intestinal cell model that can be used for evaluating drug absorption and drug metabolism simultaneously is required. Caco-2 cells have been used as such a cell model, but have a problem of insufficient expression of a metabolizing enzyme. Alternatively, primary human small intestinal epithelial cells can be used as the cell model, but this is not realistic due to difficulty in obtaining and culturing these cells. Under this background, an object of the present invention is to provide means for producing, from pluripotent stem cells, enterocytes having functions corresponding to those of actual enterocytes.

Solution to Problem

The present inventors have made earnest studies to solve the above-described problems, resulting in finding that enterocytes showing high maturity and corresponding to actual enterocytes can be obtained by performing late stage culture on a collagen vitrigel (Registered trademark) membrane (CVM) in a culture method for differentiating pluripotent stem cells into enterocytes. Besides, the present inventors have found that enterocytes having high maturity can be obtained by culturing enterocytes in mature stage (for example, on or after day 15 after starting differentiation) in a medium containing BIO, HGF, dexamethasone, and calcitriol, or in a medium containing BIO, dimethyl sulfoxide, dexamethasone, and calcitriol. The present invention was accomplished based on these findings.

Specifically, the present invention provides the following [1] to [10]:

[1] An enterocyte differentiation medium, containing a GSK3 inhibitor, and at least one selected from the group consisting of an activator of a hepatocyte growth factor receptor, an adrenal cortex hormone, calcitriol, and dimethyl sulfoxide.
[2] The enterocyte differentiation medium according to [1], in which the GSK3 inhibitor is BIO.
[3] The enterocyte differentiation medium according to [1] or [2], in which the activator of a hepatocyte growth factor receptor is HGF.
[4] The enterocyte differentiation medium according to any one of [1] to [3], in which the adrenal cortex hormone is dexamethasone.
[5] A method for producing enterocytes, including the following steps (1) to (3): (1) culturing pluripotent stem cells in a medium containing an activator of activin receptor-like kinase-4,7; (2) culturing the cells resulting from the step (1) in a medium containing a GSK3 inhibitor and a $\gamma$-secretase inhibitor; and (3) culturing the cells resulting from the step (2) on a high density collagen gel membrane in the enterocyte differentiation medium according to any one of [1] to [4], to obtain enterocytes.
[6] The method for producing enterocytes according to [5], in which the cells resulting from the step (1) are cultured on a high density collagen gel membrane in the step (2).
[7] The method for producing enterocytes according to [5] or [6], in which the pluripotent stem cells are cultured on M15 cells in the step (1).
[8] The method for producing enterocytes according to any one of [5] to [7], in which the activator of activin receptor-like kinase-4,7 is activin A in the step (1) .
[9] The method for producing enterocytes according to any one of [5] to [8], in which the GSK3 inhibitor is BIO, and the $\gamma$-secretase inhibitor is DAPT in the step (2) .
[10] Enterocytes produced by the method according to any one of [5] to [9].

This application is based upon and claims the benefit of priority of Japanese Patent Applications (Japanese Patent Application Nos. 2018-168957 and 2019-117748), the entire contents of which are incorporated herein by reference.

Advantageous Effects of Invention

The present invention provides enterocytes having high maturity, and a medium and a method to be employed for producing the same. The enterocytes having high maturity can be used as a cell model for pharmacokinetic studies.

Brief Description of Drawings

[Figure 1] Collagen vitrigel supports the differentiation of human iPSCs into enterocytes. ChiPS18 or RPChiPS771 human iPS cells differentiate into enterocytes characterized by their membrane formation and intestinal marker expression on a CVM cell culture insert (hereinafter abbreviated as CVM insert). Figure 1(A) is a schematic diagram of differentiation procedure of hiPSCs for inducing differentiation into enterocytes. Differentiation into definitive endoderm (DE) is done by culturing hiPSCs on M15 feeders with M1 media until day 3 (D3), and differentiation into intestinal precursor cells is done by continuous culture with M2 media from day 3 until day 10 (D10), or day 15 (D15). The DE cells on day 3 (D3), the intestinal precursor cells on day 10 (D10) or day 15 (D15) are dissociated and frozen, and are named as D3 (a), D10 (b) or D15 (c) stocks. Upon use, the cryopreserved cell stocks are freeze thawed and plated onto a CVM for continuing differentiation. The resultants are cultured on a CVM, the medium is switched to M2 medium on day 3, and the resultant is cultured until day 15, then the medium is further switched to M3 medium on day 15, and the resultant is cultured until day 20 or day 40. Figure 1(B) illustrates expression of an endodermal marker SOX17 (red) on day 4, and an intestinal marker CDX2 (green) on day 10. DAPI (blue) shows nuclei counter staining. Figure 1(C) illustrates TEER (transepithelial electrical resistance) values of Caco-2 cells (left) and iPSC-derived intestine (right) from the day 3, 10 or 15 stocks, and these values increase with increasing days of differentiation, and reach plateaus after performing culture for certain days. Figure 1(D) illustrates ZO1 (green) expression of iPSC-derived enterocytes. Figure 1(E) illustrates VILLIN (red) expression observed to localize on an apical side of the hiPSC- enterocytes. This drawing illustrates the Z-stacks of images obtained by scanning in the Z-axis direction on I (green line) and II (red line) planes in a cross-section showed in a box area.

[Figure 2] Figure 2 illustrates time dependent expression of transporters and P450 enzymes in the hiPSC-derived enterocytes. Human iPSC differentiate and show characteristics of the enterocytes such as expressions of intestinal markers, transporters and CYP enzymes. Figure 2(A) is a schematic diagram of the differentiation procedure in which hiPSCs are differentiated into DE cells on day 3, then replated onto a CVM, and cultured until day 40. Figure 2(B) illustrates time dependent expression of CDX2 (an intestinal precursor marker), VILLIN1 (an enterocyte marker), and LGR5 (an intestinal stem cell marker). LGR5 is transiently upregulated early but downregulated later in differentiation, when increase in CDX2 and VILLIN1 expression is observed. Figure 2(C) illustrates that ABCB1, ABCG2 and SLC15A1 transporter expression is upregulated from day 10 or day 15 of differentiation, and the level is comparable to that of the adult intestine (= 1). Figure 2(D) illustrates that CYP2C9, CYP2C19 and CYP3A4 are upregulated from day 20.

[Figure 3] Figure 3 illustrates that intestinal precursor cells differentiate into mature enterocytes on a CVM insert. Figure 3(A) is a schematic diagram of the experimental design. hiPSC-derived cryopreserved cell stocks of day 3 (D3), day 10 (D10) and day 15 (D15) are plated onto CVM inserts, and cultured until day 35 or day 40. Figure 3(B) illustrates that the day 10 or day 15 stock differentiate, and give rise to cells expressing CDX2 and VILLIN at a higher level than those of intestinal cell lines obtained from the day 3 cryopreserved DE cell stock. LGR5 expression is low in all samples. Figure 3(C) illustrates that all cryopreserved cell stocks give rise to differentiated cells expressing ABCB1, ABCG2 and SLC15A1. Figure 3(D) illustrates that the day 10 or 15 cryopreserved cell stock give rise to enterocytes expressing CYP2C9 or CYP2C19 at higher levels than enterocytes obtained from the day 3 cryopreserved DE cell stock. All cryopreserved cell stocks give rise to enterocytes expressing CYP3A4. The CYP3A4 expression level of enterocytes obtained from the day 10 cell stock is similar to the CYP3A4 expression level of enterocytes obtained from the day 3 cell stock, but the CYP3A4 expression level of enterocytes obtained from the day 15 cell stock is lower than the CYP3A4 expression level of enterocytes obtained from the day 3 cell stock. Data is expressed as a mean ± SEM. N = 3. A difference between enterocytes derived from the day 3 cryopreserved cell stock and enterocytes derived from the day 10 cryopreserved cell stock, and a difference between enterocyte cells derived from the day 3 cryopreserved cell stock and enterocytes derived from the day 15 cryopreserved cell stock are analyzed by Student's t-test. Significance is shown as *$P < 0.05$ or **$P < 0.01$.

[Figure 4] Figure 4 illustrates that hiPSC-derived enterocytes show transporter expression or CYP enzyme expression at a higher level than the Caco-2 cells. Figure 4(A) is a schematic diagram of the experimental design. The Caco-2 cells are used as a control. Day 3 cryopreserved DE cell stocks of RPChiPS771 and ChiPS18 hiPSCs are differentiated until day 21, and the expression levels of molecular markers are compared with those of the Caco-2 cells. Figure 4(B) illustrates that hiPSC-derived enterocytes express CDX2 and VILLIN at higher levels but LGR5 at a lower level as compared with the Caco-2 cells. Figure 4(C) illustrates that hiPSC-derived enterocytes express ABCB1, ABCG2 and SLC15A1 at higher levels as compared with the Caco-2 cells. Figure 4(D) illustrates that hiPSC-derived enterocytes express CYP2C9, CYP2C19 and CYP3A4. CYP3A4 is not expressed in the Caco-2 cells. Differences between enterocytes derived from RPChiPS771 and ChiPS18 hiPSCs are analyzed by Student's t-test. Significance is shown as $^*P < 0.05$ or $^{**}P < 0.01$.

[Figure 5] Figure 5 illustrates that hiPSC-derived enterocytes show functions of efflux transporters. The activities of the efflux transporters (P-gp, BCRP) are examined with human iPSC-derived enterocytes. Figure 5(A) illustrates transport activities of P-gp and BCRP tested with their substrates and inhibitors. (Left panel) Directional transcellular transport (B-to-A transport and A-to-B transport) of digoxin (a selective substrate of P-gp) in the absence or presence of verapamil (an inhibitor of P-gp) is illustrated. (Middle panel) Directional transcellular transport (B-to-A transport and A-to-B transport) of prazosin (a substrate of BCRP and P-gp) in the absence or presence of elacridar (a dual inhibitor of P-gp and BCRP) is illustrated. (Right panel) A schematic diagram of the experiment is illustrated. Directional transport of the substrate is tested by adding the substrate into an upper or lower chamber, and a drug concentration is measured on the opposite side of initial drug application. Then, absorptive (A-to-B) or secretory (B-to-A) transport of the substrate across a cell monolayer is evaluated.

Figure 5(B) illustrates examination of transport of propranolol across a cell monolayer. Propranolol is mainly transported through a transcellular route without any active transport system. Figure 5(C) illustrates examination of transport of mannitol. Mannitol is transported through a paracellular route.

[Figure 6] Figure 6 illustrates hiPSC-derived enterocytes generated under alternative differentiation protocol. Figure 6(A) is a schematic diagram of the experimental procedure for differentiating hiPS RPChiPS771 into enterocytes. Day 3 cryopreserved DE cells derived from RPChiPS771 cells differentiated on M15 are freeze thawed and plated onto a collagen vitrigel membrane (CVM), and are cultured in M2 until day 15, and then the medium is changed to M3-1 or M3-2 (a). Alternatively, a day 10 intestinal precursor stock is prepared by culturing the day 3 cryopreserved DE cells on an iMatrix coated plate until day 10, and then a day 10 cryopreserved cell stock is prepared. Upon use, the day 10 cryopreserved cell stock is freeze thawed and plated onto a CVM and cultured in M2 medium. On day 15, the medium is changed to M3-1 or M3-2, and the culture is continued until days 21 to 30.

Figure 6(B) illustrates that a TEER (transepithelial electrical resistance) value of iPSC derived intestine cultured in M3-1 or M3-2 from day 15 increases with increasing days of differentiation, and reaches a plateau after performing the culture for certain days. The intestine is obtained by differentiation from day 3 (left) or day 10 (right) cryopreserved cell stocks.

[Figure 7] Figure 7 illustrates time dependent expression of differentiation markers, transporters and P450 enzymes in the hiPSC-derived enterocytes. Human iPSCs differentiate and show expressions of intestinal markers, and transporters and CYP enzymes characteristic of the enterocytes. Figure 7(A) is a schematic diagram of the differentiation procedure of hiPSCs. RPChiPS771 cells are used. A day 3 cryopreserved DE cell stock is used. The day 3 DE cell stock is freeze thawed, plated onto a collagen vitrigel membrane (CVM), and cultured in M2 medium until day 15, then the medium is changed to M3-2 medium, and the resultant is cultured until day 30.

Figure 7(B) illustrates time dependent expression of CDX2 (an intestinal marker), VILLIN1 (an enterocyte marker), and LGR5 (an intestinal stem cell marker). LGR5 is transiently upregulated early but is downregulated later in differentiation when CDX2 and VILLIN1 expression increases. Figure 7(C) illustrates that the expression of ABCB1, ABCG2 and SLC15A1 is upregulated from day 10 or day 15 of differentiation, and the level is comparable to that of the adult intestine (= 1). Figure 7(D) illustrates that the expressions of CYP2C9, CYP2C19 and CYP3A4 are upregulated from day 15.

[Figure 8] Figure 8 illustrates that day 3 endoderm or day 10 intestinal precursor cells cryopreserved on CVM inserts differentiate into mature enterocytes. Figure 8(A) is a schematic diagram of the experimental design. Cryopreserved cell stocks of day 3 (D3) DE or day 10 intestinal precursor cells are used. The cryopreserved cells are freeze thawed and plated onto a collagen vitrigel membrane (CVM), and cultured in M2 until day 15 of differentiation. The medium is then changed to M3-1 or M3-2. The cells are cultured on a CVM insert until day 30. Figures 8(B) and 8(C) illustrate examinations of the expression of differentiation markers, transporters and metabolizing enzymes on day 21 (B) or day 30 (C). As illustrated in Figures 8(B) and 8(C), the day 3 cryopreserved DE cells differentiate and give rise to cells expressing CDX2 and VILLIN, ABCB1, ABCG2 or SLC15A1 transporter, or metabolizing enzymes CYP2C9, CYP2C19 or CYP3A4. The expression levels of the markers examined are not different between cells that are cultured in media M3-1 and M3-2. The day 10 intestinal precursor cell cryopreserved stock also differentiates into cells expressing differentiation markers. LGR5 expression is low in all samples. Data is expressed as a mean ± SEM. N = 3.

[Figure 9] Figure 9 illustrates that cryopreserved day 3 endodermal cell or day 10 intestinal precursor cell differentiates into mature enterocytes that show transporter expression or CYP enzyme expression at a higher level than the Caco-2 cells. Figure 9(A) is a schematic diagram of the experimental design. The Caco-2 cells are used as a control. A day 3 cryopreserved cell stock of RPChiPS771 is differentiated until day 21, and the resultant expression levels of molecular markers are compared with those of the Caco-2 cells. Figure 9(B) illustrates that the Caco-2 cells show TEER increasing with increasing days of differentiation, and the value reaches a plateau at about 60 $\Omega cm^2$ on day 12. Figure 9(C) illustrates that hiPSC-derived enterocytes express CDX2 and VILLIN at higher levels but LGR5 at a lower level as compared with the Caco-2 cells. Figure 9(D) illustrates that hiPSC-derived enterocytes express ABCB1, ABCG2 and SLC15A1. The expression levels are not largely different from those of the Caco-2 cells. Figure 9(E) illustrates that hiPSC-derived enterocytes express CYP2C9 and CYP2C19 at levels similar to those in the Caco-2 cells. The CYP3A4 expression is found in the hiPSC-derived enterocytes, but is not found in the Caco-2 cells.

[Figure 10] Figure 10 illustrates that hiPSC-derived enterocytes show functions of efflux transporters. Rhodamine 123 flux analysis on day 26 reveals that P-gp transporter activity in transporting its specific substrate, Rhodamine 123, in a basal to apical direction is active in the hiPS-derived cells. Figure 10(A) illustrates that human RPChiPS771 iPSC-derived enterocytes are differentiated using M3-1 instead of M3 medium. A cryopreserved day 3 DE stock (left panels) or day 10 enterocyte stock (right panels) is used. The directional transcellular transport (A-to-B and B-to-A) of Rhodamine 123 (P-gp selective substrate) is observed on day 21 (upper panels) or day 30 (lower panels) of differentiation. Figure 10(B) illustrates differentiation of human RPChiPS771 iPSC-derived enterocytes using M3-2 instead of M3 medium. The directional transcellular transport of Rhodamine 123 is observed on day 26. Values of permeabilities are shown in a table below each graph.

[Figure 11] Figure 11 illustrates that expression of CYP3A4 is induced by culture in M3-2 medium on days 23 to 25. On days 23 to 25, enterocytes cultured in M3-2 medium express CYP3A4 at a level similar to that in enterocytes cultured in M3-2 medium on 15 to 25 days.

[Figure 12] Figure 12 illustrates that calcitriol plays an important role in inducing expression of CYP3A4. Enterocytes cultured in a medium not containing calcitriol minimally express CYP3A4.

Description of Embodiments

The present invention will now be described in detail.

(A) Enterocyte Differentiation Medium

An enterocyte differentiation medium of the present invention contains a GSK3 inhibitor, and at least one selected from the group consisting of an activator of a hepatocyte growth factor receptor, an adrenal cortex hormone, calcitriol, and dimethyl sulfoxide.

In the present invention, the term "enterocyte differentiation medium" refers to a medium used for differentiating, into enterocytes, cells that have not differentiated into enterocytes. The term "cells that have not differentiated into enterocytes" refers to, for example, cells obtained in the step (2) of a method for producing enterocytes of the present invention described below, and more specifically, refer to intestinal precursor cells.

Medium components to be contained in the enterocyte differentiation medium of the present invention can be a GSK3 inhibitor, an activator of a hepatocyte growth factor receptor, an adrenal cortex hormone, calcitriol, and dimethyl sulfoxide. Among these, the GSK3 inhibitor is an essential component, and as the activator of a hepatocyte growth factor receptor, the adrenal cortex hormone, calcitriol, and dimethyl sulfoxide, at least one selected from these components may be contained in the medium. Besides, merely either one of the activator of a hepatocyte growth factor receptor and dimethyl sulfoxide may be usually contained. Furthermore, when the expression of CYP3A4 is to be induced, calcitriol is preferably contained in the medium. Examples of a preferable combination of the medium components include a combination of the GSK3 inhibitor, the adrenal cortex hormone, calcitriol, and dimethyl sulfoxide, and a combination of the GSK3 inhibitor, the adrenal cortex hormone, calcitriol, and the activator of a hepatocyte growth factor receptor.

The GSK3 inhibitor is selected from the group consisting of a substance having GSK3$\alpha$ inhibitory activity, a substance having GSK3$\beta$ inhibitory activity, and a substance having both GSK3$\alpha$ inhibitory activity and GSK3$\beta$ inhibitory activity. The GSK3 inhibitor is preferably a substance having GSK3$\beta$ inhibitory activity, or a substance having both GSK3$\alpha$ inhibitory activity and GSK3$\beta$ inhibitory activity. Specific examples of the GSK3 inhibitor include CHIR98014, CHIR99021, Kenpaullone, AR-AO144-18, TDZD-8, SB216763, BIO, TWS-119, and SB415286. These substances are commercially available. Alternatively, even when not commercially available, those skilled in the art can prepare these substances in accordance with description of known literatures. Besides, an antisense oligonucleotide, a siRNA or the like against mRNA of GSK3 can be used as the GSK3 inhibitor. All of these are commercially available, or can be synthesized in accordance with description of known literatures. The GSK3 inhibitor to be used is preferably BIO. A concentration of the GSK3 inhibitor in the medium is appropriately set in accordance with the type of the GSK3 inhibitor to be used, and when BIO is used as the GSK3 inhibitor, the concentration is preferably 0.1 to 5 $\mu$M, and more preferably 0.5 to 3 $\mu$M.

The activator of a hepatocyte growth factor receptor is not especially limited as long as it can activate a hepatocyte growth factor receptor, and an example includes HGF. A concentration of the activator of a hepatocyte growth factor receptor in the medium is appropriately set in accordance with the type of the activator of a hepatocyte growth factor receptor to be used, and when HGF is used as the activator of a hepatocyte growth factor receptor, the concentration is preferably 1 to 50 ng/mL, and more preferably 5 to 30 ng/mL.

The adrenal cortex hormone may be a chemically synthesized one, or a natural one. Specific examples of the adrenal cortex hormone include dexamethasone and hydrocortisone. The adrenal cortex hormone is preferably dexamethasone. A concentration of the adrenal cortex hormone in the medium is appropriately set in accordance with the type of the adrenal cortex hormone to be used, and when dexamethasone is used as the adrenal cortex hormone, the concentration is preferably 0.01 to 1 $\mu$M, and more preferably 0.05 to 0.3 $\mu$M.

A concentration of calcitriol in the medium is not especially limited, and is preferably 0.1 to 5 $\mu$M, and more preferably 0.5 to 3 $\mu$M.

A concentration of dimethyl sulfoxide in the medium is not especially limited, and is preferably 0.1 to 1%, and more preferably 0.3 to 0.8%.

The enterocyte differentiation medium of the present invention may contain another component in addition to the GSK3 inhibitor, the activator of a hepatocyte growth factor receptor, the adrenal cortex hormone, calcitriol, and dimethyl sulfoxide. For example, ascorbic acid, bovine serum albumin (preferably fatty acid free), transferrin, insulin, L-glutamine or the like may be contained.

The enterocyte differentiation medium of the present invention can be produced based on a known basal medium. Examples of the known basal medium include BME medium, BGjB medium, CMRL 1066 medium, Glasgow MEM medium, Improved MEM medium, IMDM medium, Medium 199 medium, Eagles MEM medium, $\alpha$MEM medium DMEM medium, Ham's medium, RPMI 1640 medium, Fischer's medium, William's E medium, and a mixed medium of any of these, but the medium is not especially limited as long as it can be used for culturing animal cells.

The enterocyte differentiation medium of the present invention may contain a serum substitute. Examples of the serum substitute include albumin, transferrin, fatty acid, a collagen precursor, a trace element (such as selenium), B-27 supplement, E5 supplement, E6 supplement, N2 supplement, knockout serum replacement (KSR), 2-mercaptoethanol, 3'-thiolglycerol, and an equivalent of any of these. These serum substitutes are commercially available. Preferably, a Xeno-free B-27 supplement or Xeno-free knockout serum replacement (KSR) can be used to be added to the medium at a concentration of, for example, 0.01 to 30% by weight, and preferably 0.1 to 20% by weight. Besides, the medium may contain another additive such as a lipid, an amino acid (such as a nonessential amino acid), a vitamin, a growth factor, a cytokine, an antioxidant, 2-mercaptoethanol, pyruvic acid, a buffer, an inorganic salt, an antibiotic (such as penicillin or streptomycin), or an antibacterial agent (such as amphotericin B).

(B) Method for Producing Enterocytes

A method for producing enterocytes of the present invention includes the following steps (1) to (3).

In the step (1), pluripotent stem cells are cultured in a medium containing an activator of activin receptor-like kinase-4,7.

The term "pluripotent stem cells" refers to cells that have self-replicating ability, can be cultured *in vitro,* and have pluripotency to be able to differentiate into cells constituting an individual. Specific examples include embryonic stem cells (ES cells), pluripotent stem cells derived from primordial germ cells of an embryo (GS cells), induced pluripotent stem cells derived from somatic cells (iPS cells), and somatic stem cells. In the present invention, iPS cells or ES cells are preferably used, and human iPS cells or human ES cells are more preferably used.

ES cells are preferably ES cells derived from a mammal. Examples of the mammal include a mouse, a rat, a guinea pig, a hamster, a rabbit, a cat, a dog, a sheep, a cow, a horse, a goat, a monkey, or a human, and the mammal is preferably a mouse or a human, and more preferably a human.

For obtaining ES cells, a fertilized egg at the blastocyst stage is cultured together with feeder cells, an operation for scattering the thus grown cells derived from an internal cell mass and subculturing the resultant cells is repeated, and ultimately, ES cells can be established as a cell line.

Besides, iPS cells refer to cells having acquired differentiation pluripotency, and cells that have acquired differentiation pluripotency similar to that of ES cells by introducing several types of transcription factors (pluripotency factors) for imparting differentiation pluripotency to somatic cells (such as fibroblasts). As the "pluripotency factors", a large number of factors have been reported, and examples include, but are not limited to, members of Oct family (such as Oct3/4), Sox family (such as Sox2, Sox1, Sox3, Sox15, and Sox17), Klf family (such as Klf4, and Klf2), Myc family (such as c-Myc, N-Myc, and L-Myc), Nanog, and LIN28. There are a large number of reports on a method for establishing iPS cells, which can be referred to (for example, Takahashi et al., Cell, 2006, 126:663-676; Okita et al., Nature, 2007, 448:313-317; Wernig et al., Nature, 2007, 448:318-324; Maherali et al., Cell Stem Cell, 2007, 1:55-70; Park et al., Nature, 2007, 451:141-146; Nakagawa et al., Nat Biotechnol 2008, 26:101-106; Wernig et al., Cell Stem Cell, 2008, 10:10-12; Yu et al., Science, 2007, 318:1917-1920; Takahashi et al., Cell, 2007, 131:861-872; and Stadtfeld et al., Science, 2008, 322:945-949).

The pluripotent stem cells to be used can be cultured and maintained by any method usually employed in this field. A method for culturing the ES cells can be performed by an ordinary method. For example, the ES cells can be maintained by using mouse embryo fibroblasts (MEF cells) as feeder cells, and by using a medium containing a leukemia inhibitory factor, KSR (knockout serum substitute), fetal bovine serum (FBS), nonessential amino acid, L-glutamine, pyruvic acid, penicillin, streptomycin, and β-mercaptoethanol, such as DMEM medium. Also the iPS cells can be cultured by an ordinary method. For example, the iPS cells can be maintained by using MEF cells as feeder cells, and by using a medium containing bFGF, KSR (knockout serum substitute), nonessential amino acid, L-glutamine, penicillin, streptomycin, and β-mercaptoethanol, such as DMEM/F12 medium.

The pluripotent stem cells may be cultured, before the culture performed in the step (1), in an undifferentiation-maintaining medium not containing methionine by a known method (WO2015/125662). Thus, differentiation efficiency of the pluripotent stem cells can be accelerated. Here, the term "not to contain methionine" means not only that the medium contains no methionine but also that the medium contains 10 μM or less, preferably 5 μM or less, more preferably 1 μM or less, and further preferably 0.1 μM or less of methionine. Culture time in the medium not containing methionine is not especially limited, and is preferably 3 to 24 hours, more preferably at least 5 to 24 hours, and further preferably at least 5 to 10 hours.

The activator of activin receptor-like kinase (ALK)-4,7 is selected from substances having an action to activate ALK-4 and/or ALK-7. Examples of the activator of activin receptor-like kinase-4,7 to be used include activin, Nodal, and Myostatin, and activin is preferred. As activin, activins A, B, C, D and AB are known, any of which activin can be used. Activin to be used is particularly preferably activin A. Besides, as activin to be used, activin derived from any of mammals such as a human and a mouse can be used, and activin derived from the same animal as that of stem cells used for the differentiation is preferably used. For example, when pluripotent stem cells derived from a human are used as the starting material, activin derived from a human, particularly, activin A derived from a human is preferably used. These activins are commercially available. A concentration of the activator of activin receptor-like kinase-4,7 in the medium is appropriately set in accordance with the type thereof to be used, and when human activin A is used, the concentration is preferably 3 to 500 ng/mL, and more preferably 5 to 200 ng/mL.

In the medium, a GSK3 inhibitor may be contained in addition to the activator of activin receptor-like kinase-4,7. Besides, with the culture performed in two stages, a medium containing the activator of an activin receptor-like kinase-4,7 and the GSK3 inhibitor may be used in the first stage, and a medium containing the activator of activin receptor-like kinase-4,7 but not containing the GSK3 inhibitor may be used in the second stage.

The same GSK3 inhibitors as those used in the enterocyte differentiation medium of the present invention can be used, and the GSK3 inhibitor suitably used in the step (1) is selected from the group consisting of CHIR99021 (6-[[2-[[4-(2,4-dichlorophenyl)-5-(4-methyl-1H-imidazol-2-yl)-2-pyrimidinyl]amino]ethyl]amino]nicotinonitrile), SB216763 (3-(2,3-dichlorophenyl)-4-(1-methyl-1H-indol-3-yl)-1H-pyrrole-2,5-dione), and SB415286 (3-[(3-chloro-4-hydroxyphenyl)amino]-4-(2-nitrophenyl)-1H-pyrrole-2, 5-dione), and CHIR99021 is particularly preferred. A concentration of the GSK3 inhibitor in the medium is appropriately set in accordance with the type of the GSK3 inhibitor to be used, and when CHIR99021 is used as the GSK3 inhibitor, the concentration is preferably 1 to 10 μM, and more preferably 2 to 5 μM.

The medium to be used in the step (1) can be produced based on a known basal medium. A basal medium similar to that used for the enterocyte differentiation medium of the present invention can be used. Besides, the medium to be used in the step (1) may contain a serum substitute. A serum substitute similar to that used in the enterocyte differentiation medium of the present invention can be used. Furthermore, the medium to be used in the step (1) may contain the same additives as those used in the enterocyte differentiation medium of the present invention.

The culture of the step (1) is preferably performed on M15 feeder cells. Thus, the pluripotent stem cells can be efficiently induced into endodermal cells (Shiraki et al., 2008, Stem Cells 26, 874-85).

The culture of the step (1) is performed usually in a $CO_2$ incubator at a culture temperature suitable for cell culture (usually 30 to 40°C, preferably about 37°C). A culture period is usually 1 to 5 days, and preferably 3 to 4 days. Besides, the culture period may be determined by checking whether or not the pluripotent stem cells under cultivation have differentiated into definitive endodermal cells. It can be checked, by evaluating differential expression of proteins or genes (endoderm markers) expressing specifically to endodermal cells, whether or not the pluripotent stem cells have differentiated into definitive endodermal cells. The evaluation of differential expression of an endoderm marker can be performed by, for example, an expression evaluation method for a protein utilizing an antigen-antibody reaction, or a gene expression evaluation method utilizing quantitative RT-PCR. Examples of the endoderm markers include SOX17 and FOXA2.

In the step (2), the cells obtained in the step (1) are cultured in a medium containing a GSK3 inhibitor and a γ-secretase inhibitor.

The culture of the step (2) may be performed immediately after the culture of the step (1), or the cells may be cryopreserved after completing the step (1), and thawed after storage of a certain period to be subjected to the culture of the step (2).

The same GSK3 inhibitors as those used in the enterocyte differentiation medium of the present invention can be used, and BIO can be used as a suitable GSK3 inhibitor. A concentration of the GSK3 inhibitor in the medium is appropriately set in accordance with the type of the GSK3 inhibitor to be used, and when BIO is used as the GSK3 inhibitor, the concentration is preferably 0.5 to 20 μM, and more preferably 1 to 10 μM.

Examples of the γ-secretase inhibitor include arylsulfonamide, dibenzazepine, benzodiazepine, DAPT, L-685458, and MK0752. These are commercially available. Alternatively, even when not commercially available, those skilled in the art can prepare these in accordance with description of known literatures. A suitable example of the γ-secretase inhibitor includes DAPT. A concentration of the γ-secretase inhibitor in the medium is appropriately set in accordance with the type of the γ-secretase inhibitor to be used, and when DAPT is used as the γ-secretase inhibitor, the concentration is preferably 1 to 30 μM, and more preferably 5 to 20 μM.

The culture of the step (2) is performed preferably on M15 feeder cells or a high density collagen gel membrane. Alternatively, the culture of the step (2) may be performed on M15 feeder cells at a first stage, and on a high density collagen gel membrane at a second stage.

The term "high density collagen gel" refers to collagen gel containing high density collagen fiber comparable to biological connective tissue, and it is excellent in strength and transparency. An example of the high density collagen gel includes collagen vitrigel (registered trademark of National Agriculture and Food Research Organization) (Toshiaki Takezawa: The Society for Biotechnology Brochure 91: 214-217, 2013, WO2012/026531, Japanese Patent Laid-Open No. 2012-115262). The high density collagen gel can be produced by causing collagen sol to undergo gelation, drying the resultant collagen gel for vitrification, and then rehydrating the resultant. A collagen fiber density in the high density collagen gel is preferably 21 to 45% (W/V), more preferably 26 to 40% (W/V), and further preferably 31 to 35% (W/V).

The culture on the high density collagen gel membrane may be performed by any method, and it is preferable that the high density collagen gel membrane be provided on a bottom of a cylindrical frame so as to culture the cells on this membrane.

The medium used in the step (2) can be produced based on a known basal medium. A basal medium similar to that used for the enterocyte differentiation medium of the present invention can be used. Besides, the medium used in the step (2) may contain a serum substitute. As the serum substitute, one similar to that used in the enterocyte differentiation medium of the present invention can be used. A concentration of the serum substitute in the medium is not especially limited, and can be, for example, 0.01 to 30% by weight, and preferably 0.1 to 20% by weight. Furthermore, the medium used in the step (2) may contain the same additives as those used in a medium used as the enterocyte differentiation medium of the present invention.

The culture of the step (2) is performed usually in a $CO_2$ incubator at a culture temperature suitable for cell culture (usually 30 to 40°C, preferably about 37°C). A culture period is usually 8 to 20 days, and preferably 10 to 15 days. Besides, the culture period may be determined by checking whether or not the cells under cultivation have differentiated into intestinal precursor cells. The differentiation into intestinal precursor cells can be checked by evaluating differential expression of proteins or genes (intestinal precursor cell markers) expressing specifically to intestinal precursor cells. The evaluation of differential expression of an intestinal precursor cell marker can be performed by, for example, an expression evaluation method for a protein utilizing an antigen-antibody reaction, or a gene expression evaluation method utilizing quantitative RT-PCR. Examples of the intestinal precursor markers include CDX2 and IFABP.

In the step (3), the cells obtained in the step (2) are cultured on a high density collagen gel membrane in the enterocyte differentiation medium of the present invention to obtain enterocytes.

The culture of the step (3) may be performed immediately after the culture of the step (2), or the cells may be cryopreserved after completing the step (2), and thawed after storage of a certain period to be subjected to the culture of the step (3).

The culture on a high density collagen gel membrane can be performed in the same manner as the culture of the step (2).

In the step (3), the enterocyte differentiation medium of the present invention is used, and another medium can be used as long as enterocytes can be obtained. An example of such another medium includes a medium including Cellaritis (Registered trademark) Hepatocyte Maintenance Medium.

For producing enterocytes expressing a high level of CYP3A4, the culture is performed preferably in a medium containing calcitriol in a second half of the step (3). On the other hand, in a first half of the step (3), it is not always necessary to perform the culture in a medium containing calcitriol. Accordingly, different media may be used in the first half and the second half of the step (3). In other words, it is possible to use the enterocyte differentiation medium of the present invention not containing calcitriol in the first half of the step (3) and to use the enterocyte differentiation medium of the present invention containing calcitriol in the second half.

The culture of the step (3) is performed usually in a $CO_2$ incubator at a culture temperature suitable for cell culture (usually 30 to 40°C, preferably about 37°C). A culture period is usually 3 to 40 days, and preferably 5 to 30 days. Besides, the culture period may be determined by checking whether or not the cells under cultivation have differentiated into enterocytes. The differentiation into enterocytes can be checked by evaluating differential expression of proteins or genes (enterocyte markers) expressing specifically to enterocytes. The evaluation of differential expression of an enterocyte marker can be performed by, for example, an expression evaluation method for a protein utilizing an antigen-antibody reaction, or a gene expression evaluation method utilizing quantitative RT-PCR. An example of the enterocyte markers includes VILLIN.

The enterocytes produced by the method for producing enterocytes described above are also the subject matter of the present invention. The enterocytes are specified not by the structure or the characteristics but by the production method, and this is for the following reason: Enterocytes are a part of a living body, and hence have extremely complicated structure and characteristics, and an operation for specifying these requires extremely large economic expenditure and time.

The enterocytes of the present invention have, for example, the following characteristics:

a) The enterocytes express ZO1, that is, a tight junction-related protein, at the cell junction.
b) The enterocytes form a monolayer. It is confirmed by the measurement of TEER values that the monolayer has a sufficient barrier function.
c) Uptake transporter genes (such as SLC15A1) and efflux transporter genes (such as ABCB1 and ABCG2) are expressed at levels similar to those in the adult intestine.
d) Transcellular directional transport of digoxin and prazosin from a basal side to an apical side is observed.
e) Gene expression of CYP enzymes (such as CYP2C9, CYP2C19, and CYP3A4) is at levels corresponding to those in a normal adult.
f) The enterocytes express VILLIN, and the expression is localized on the apical side.

EXAMPLES

Now, the present invention will be described in more details with reference to examples, and it is noted that the present invention is not limited to these examples.

(A) Results

(1) Collagen vitrigel supported the differentiation of human iPSCs into enterocytes characterized by their membrane formation and intestinal marker expression.

In an attempt to induce differentiation of human iPS cells into mature enterocytes, the present inventors first induced definitive endoderm (DE) cells from human iPSCs on M15 cells. Day 3 (D3) DE cells were dissociated, replated on a CVM insert, and cultured in M2 medium until day 15. Thereafter, the medium was changed to a maturation (M3, M3-1 or M3-2) medium, and the cells were cultured up to day 40 (Figures 1A and 6A). Alternatively, the day 3 DE cells were re-plated onto an iMatrix511 pre-coated plate and cultured until day 10 in M2 medium, and then cryopreserved as a day 10 intestinal precursor cell stock. Upon use, the cryopreserved day 10 cell stock was freeze thawed and seeded onto a CVM insert and cultured in M2 until day 15, and then the medium was changed to M3-1 or M3-2 medium. The day 3 DE cells could also be saved as a frozen cell stock. Either the day 3 DE cell stock or the day 10 intestinal precursor cell stock was used in experiments of the present inventors. As a result of immunocytochemical analysis, it was revealed that DE cells show SOX17 expression on day 4 after being plated on the CVM, and that CDX2 expression is increased on day 10 (Figure 1B).

Subsequently, in order to check the integrity of the membrane, a transepithelial electrical resistance (TEER) value was assayed. The Caco-2 cells, that is, a colon cancer derived cell line, were seeded at a concentration of $5.0 \times 10^4$ cells/well. The TEER value increased with time, and reached 35 $\Omega cm^2$ on day 14. The cryopreserved day 3 (D3) DE cells were seeded at a concentration of $8.0 \times 10^4$ cells/well, and caused to form a monolayer. In these cells, the TEER value increased with time and reached a plateau at about 40 $\Omega cm^2$ on day 14. Cryopreserved day 10 and day 15 intestinal precursor cells were also seeded at a concentration of $1.6 \times 10^5$ cells/well, and caused to form a monolayer. In these cells, the TEER value increased with time, and reached a plateau at a level of about 50 $\Omega cm^2$ 11 days after plating (Figure 1C).

The enterocytes expressed ZO1, that is, a tight junction-related protein, at the cell junction. This result suggests that the barrier function of the cells is formed (Figure 1D). The expression of VILLIN, that is, an enterocyte specific marker, was observed. The VILLIN expression showed a polarized localization, with higher expression on the apical side than on the basal side of the human iPSC-derived enterocytes. This result was revealed by confocal microscopy examination on day 30 of differentiation (Figure 1E).

These results indicate that hiPSC-derived enterocytes show marker expression, membrane integrity, and polarized apical-to-basal structure (all of which are characteristics of enterocytes).

The TEER values of the cells were examined (Figure 6). The differentiated cells were increased in the TEER upon freeze thaw, and the TEER reached to a plateau at 40 $\Omega cm^2$ on day 14 in both the day 3 (left in Figure 6) and the day 10 (right in Figure 6) cryopreserved cell stocks. Therefore, M3 medium could be substitute with M3-1 or M3-2 medium.

(2) hiPSC-derived enterocytes expressed transporters and P450 enzymes characteristics of the enterocytes.

Next, the expression of intestinal markers, transporters and metabolizing enzymes was analyzed by quantitative PCR analysis. In this set of experiments, cryopreserved day 3 DE cells were used. The cryopreserved day 3 DE cells were plated onto a CVM insert, and cultured first in M2 medium, then the medium was changed to M3 medium, and the cells were cultured up to day 40 of differentiation and thus, intestine differentiation was induced (Figure 2A).

Q-PCR analysis was performed to reveal that LGR5, that is, a marker for crypt base columnar cells, was upregulated transiently, reached a peak on day 5, and then was downregulated thereafter. With the decrease in LGR5 expression, the expression of the intestinal precursor marker CDX2 and the enterocyte marker VILLIN were upregulated in a mutual exclusive manner to the LGR5 expression. The CDX2 and VILLIN expression reached plateaus on day 10 and day 15, respectively (Figures 2A and 2B), and their expression levels were maintained at substantial levels. The expression levels of the markers were normalized to those of the adult intestine (adult intestine = 1).

Drug efflux transporters expressed in the intestine, such as BCRP (breast cancer resistance protein) encoded by ABCG2 (ATP-binding cassette family G member 2), and P-gp/MDR1 (P-glycoprotein/multidrug resistance 1) encoded by ABCB1 (ATP-binding cassette family B member 1), are considered to be a limiting barrier against drug absorption in the intestine (Estudante et al., (2013) Adv. Drug Deliv. Rev. 65, 1340-1356). PEPT1 (peptide transporter 1) encoded by SLC15A1 (solute carrier family 15 member 1), is involved in the intestinal uptake of oligopeptides and peptide-mimetic drugs. Since these transporters are important for determining the intestinal availability of orally-administered drugs, their culture period-dependent expression patterns during the differentiation from hiPSCs were checked. The mRNA expression of ABCG2 was observed to be upregulated from day 10 of differentiation, and reached a peak on day 15, and was maintained at a certain level thereafter. ABCB1 was upregulated from day 15, and reached a peak on day 30. SLC15A1 was detected from day 15, was gradually upregulated thereafter, and reached a plateau on day 30. As a whole, the transporter expression was detected on or after day 21 at a certain level (Figure 2C).

Drug metabolism is also an important function of the enterocytes for detoxification of xenobiotics. Among cytochrome P450 (CYP) isoforms, CYP3A4 is a dominant drug metabolizing enzyme in the small intestine. The mRNA expression of CYP3A4, CYP2C9 and CYP2C19 was observed to be upregulated from day 20 and increased thereafter in the hiPSC-derived enterocytes (Figure 2D). The expression levels of CYP enzymes in the hiPSC-derived intestine are shown as fold change from those in the adult intestine. The results therefore indicate that the levels of CYP2C9 and CYP2C19 are approximately 0.1 to 0.3 fold higher than those of the endogenous enzymes in the adult intestine. CYP3A4 was expressed at a level about 0.08 to 0.15 fold higher than that in the adult intestine from day 30 to day 40 of differentiation. Similar results were obtained when M3 medium was replaced with M3-1 or M3-2 medium (Figures 7 and 9).

(3) A CVM supported intestinal precursor cells to differentiate into mature enterocytes.

Next, the differentiation potential of the hiPSC-derived intestinal precursor cells was tested. In this experiment, cryopreserved day 3 DE cells, and day 10 and day 15 hiPSC-derived intestinal precursor cells were used. These cells were seeded onto CVM inserts, cultured until day 35 or 40, and then harvested and analyzed for intestinal markers, transporters and CYP enzymes by real time PCR (Figure 3A). The day 10 and day 15 hiPSC-derived intestinal precursor cells differentiated and showed low levels of LGR5 expression. The day 3 DE cells and the day 10 intestinal precursor cells gave rise to enterocytes expressing CDX2 and VILLIN at similar levels on day 35. However, day 40 enterocytes derived from the day 15 intestinal precursor cells expressed CDX2 or VILLIN at a higher level than those derived from the day 3 DE cells (Figure 3B). The results suggest that cryopreserved cell stocks of day 10 and day 15 hiPSC-derived intestinal precursor cells could differentiate into enterocytes on the CVM.

Subsequently, the expression levels of the transporter molecules of MDR1, BCRP or PEPT1, or CYP enzyme molecules of CYP2C9, CYP2C19 or CYP3A4 were tested. As a result, the cryopreserved cell stock of the day 10 hiPSC-derived intestinal precursor cells differentiated into cells expressing ABCB1 and ABCG2 at levels similar to those derived from the day 3 DE cells. On the other hand, although day 15 hiPSC-derived intestinal precursor cells gave rise to enterocytes, the expressions of ABCB1 and ABCG2 were lower in these enterocytes than in the cells derived from the day 3 DE cells (Figure 3C). However, the expression of SLC15A1 showed different tendency in these hiPSC-derived cells. The day 10 cryopreserved cells gave rise to enterocytes showing higher SLC15A1 expression than the 3 day DE cells, but 15 day cryopreserved cells had differentiation potential similar to that of the day 3 DE cells. As for the CYP enzyme expression, the 10 day hiPSC-derived intestinal precursor cells differentiated and expressed CYP3A4 at a level similar to that of the day 3 DE cells. The day 15 hiPSC-derived intestinal precursor cells gave rise to enterocytes showing lower CYP3A4 expression. However, the day 10 and day 15 hiPSC-derived intestinal precursor cells gave rise to enterocytes expressing CYP2C9 or CYP2C19 at a higher level as compared with that of the day 3 DE cells. As the expression level is normalized by setting that in the adult intestine to 1, the results suggest that the differentiated enterocytes express appropriate levels of the CYP molecules that are expressed in the intestine. Similar results were obtained when M3-1 or M3-2 medium was used instead of M3 medium (Figure 9). Cryopreserved day 10 cell stock prepared from day 3 stock using iMatrix could be also used.

(4) hiPSC-derived enterocytes showed mRNA expression of transporters comparable to that of the Caco-2 cells, and CYP3A4 enzyme expression that was not expressed in the Caco-2 cells.

The results indicate that hiPSC-derived enterocytes express intestinal markers, transporters and CYP enzymes that resemble those of the enterocytes. Next, the expression levels of the markers in the hiPSC-derived enterocytes were compared with the expression levels of the Caco-2 cells. The Caco-2 cells are the most commonly-used human intestinal cell model for evaluating the intestinal absorption property of drugs in drug development process. The Caco-2 cells were plated onto a CVM 24-well insert and cultured for 21 days. Day 3 DE cells derived from two different hiPSC lines (RPChiPS771 and ChiPS18) were plated onto CVMs and cultured for 18 days (21 days of differentiation) to examine molecular marker expression (Figure 4A).

The results show that the Caco-2 express a substantial level of LGR5, which is not observed in hiPSC-derived enterocytes. The expression levels of CDX2 and VILLIN were higher in hiPSC-derived enterocytes as compared with those expressed in the Caco-2 cells (Figure 4B). The results therefore suggest that the Caco-2 cells resemble immature cells of the intestine.

Caco-2 cultured for 21 days expressed a high level of ABCB1 as compared with the level of the adult enterocytes (Figure 9D). hiPSC-derived enterocytes on day 21 of differentiation expressed ABCB1, ABCG2 and SLC15A1 at levels comparable to those of the adult intestine (Figure 9D). The present inventors confirmed that the Caco-2 cells do not express CYP3A4. By contrast, CYP3A4 expression started to be detected in the hiPSC-derived enterocytes (Figure 4D) (see also Figure 2D, Figure 7D, Figure 8C, and Figure 9E). CYP2C9 and CYP2C19 expression was also detected (Figure 4D) (Figure 7D, Figure 8C, and Figure 9E).

Results obtained by the present inventors therefore indicate that hiPSC cultured on a CVM insert can differentiate and generate enterocytes. The thus generated enterocytes showed expressions of mature intestinal markers, transporters and CYP enzymes at levels higher than those expressed in the Caco-2 cells. Similar results were obtained when M3-1 or M3-2 medium was used instead of M3 medium. A cryopreserved day 10 cell stock prepared from a day 3 stock using iMatrix could be also used.

(5) hiPSC-derived enterocytes were observed to exert the active efflux transport and CYP3A4-mediated metabolism of drugs.

Since ABCG2 and ABCB1 mRNAs are highly expressed in the hiPSC-derived enterocytes, a bidirectional transcellular transport assay was performed to examine the transport activities of efflux transporters (P-gp, BCRP) in the enterocytes on a cell culture insert (right in Figure 5A). Directional basal-to-apical transport (B-to-A transport) of digoxin, that is, a typical substrate of P-gp, was observed in a time-dependent manner at a level higher than apical-to-basal transport (A-to-B transport) (left panel in Figure 5A). On the other hand, in the presence of verapamil, that is, a typical inhibitor of P-gp, the B-to-A transport of digoxin was almost the same as the A-to-B transport. Similarly, the B-to-A transport of prazosin, that is, a substrate of BCRP and P-gp, was found to be higher than the A-to-B transport. In the presence of elacridar, that is, an inhibitor of both BCRP and P-gp, the B-to-A transport of prazosin was decreased but the A-to-B transport was increased. These results therefore suggested that the hiPSC-derived enterocytes exhibit transport activity of the intestinal efflux transporters, P-gp and BCRP (middle panel in Figure 5A).

The present inventors also tested transport of propranolol and mannitol. The transport of propranolol is thought to be mediated by passive membrane permeation due to its high hydrophobicity. The transport of mannitol is thought to be mediated via a paracellular route due to its low molecular weight and high hydrophilicity. As a result, the directional transport of neither propranolol nor mannitol across a cell monolayer was observed (Figures 5B and 5C), and hence involvement of active transport was not found. Moreover, the transport of propranolol was much higher than that of mannitol. This is reasonably explained by their different physicochemical properties.

The present inventors also tested the metabolic activity of CYP3A4 by observing hydroxylation of midazolam to form 1'-OH midazolam. The hydroxylation of midazolam is known to be selectively driven by CYP3A4. As a result, it was found, as a result of evaluation by LC-MS/MS, that the formation of 1'-OH midazolam occurs at a clearance rate of 0.773 μL/min/mg cellular protein. In summary, the results obtained by the present inventors indicate that the enterocytes have efflux transporter activities of both P-gp and BCRP, and CYP3A4-mediated metabolic activity.

(6) Alternative Differentiation Procedure to Generate hiPS-derived Enterocytes

In the above differentiation procedure, the present inventors found that the hiPS-derived enterocytes express transporters ABCB1 at a certain level on day 15 (Figure 2C) and increase the expression of ABCG2 and SLC15A1 on and after day 15. These hiPS-derived enterocytes also started to express drug metabolizing enzymes from day 15 (Figure 2D) after being cultured in M3. In order to reveal molecules that are important for guiding maturation of precursor cells into mature enterocytes expressing the transporters and metabolizing enzymes, the present inventors examined effects of substituting M3 medium with M3-1 or M3-2 medium (Figures 6 to 10). The results revealed that when BIO, DMSO, Dexamethasone, and calcitriol (M3-2) were added to the cells, hiPS-derived enterocytes expressed the transporters and metabolizing enzymes CYP3A4 at a level similar to that in hiPS-derived enterocytes generated under culturing in the conventional M3 medium, and that the level was higher than that of the results obtained by M3-1 medium (Figures 8 and 9).

In summary, the results obtained by the present inventors showed that the presence of BIO, DMSO, dexamethasone, and calcitriol in M3-2 is important for promoting the maturation of the enterocytes for their metabolizing enzyme CYP3A4 expression (Figures 8B and 8C). The derived hiPS-enterocytes exhibited P-gp-mediated efflux transporter activity to excrete rhodamine 123 substrate.

(7) Induction of CYP3A4 Expression by Calcitriol

The expression of CYP3A4 was checked by using, instead of M3-2 medium, a medium obtained by removing calcitriol from M3-2 medium. hiPSC-derived enterocytes cultured in the medium in which calcitriol had been removed minimally expressed CYP3A4 on day 21 of differentiation (Figure 12).

Expression of CYP3A4 was checked in hiPSC-derived enterocytes cultured in a medium obtained by removing calcitriol from M3-2 medium on days 15 to 22, and subsequently cultured in M3-2 medium on days 23 to 25. The CYP3A4 expression level of the hiPSC-derived enterocytes was similar to that of cells cultured in M3-2 medium on days 15 to 25, and was higher than that of cells cultured in M3 medium (Figure 11).

These results indicate that the culture in a medium containing calcitriol at the final stage (days 23 to 25 of differentiation) of the enterocyte differentiation is important for the induction of the CYP3A4 expression.

(B) Discussion

Here, it was established that functional enterocytes are generated from hiPSCs by culturing hiPSC-derived endoderm or intestinal precursor cells on a CVM. The present inventors found that the CVM is a good substrate for the induction and maintenance of mature enterocytes. The present inventors previously reported that CDX2-positive enterocytes can be obtained by culturing hiPSCs on M15 cells and adding thereto BIO, that is, a Wnt signal activator, and DAPT, that is, a Notch signal inhibitor. The hiPSC-derived intestinal precursor cells cultured on the CVM are able to form a monolayer with the expression of VILLIN localized in the apical membrane. The differentiated cells are ZO1-positive and exhibit barrier function revealed by the TEER measurement. These features indicate that functional enterocytes are generated.

Gene expression analysis revealed that the induced enterocytes show high mRNA expression levels of the uptake transporter (SLC15A1) and the efflux transporters (ABCB1 and ABCG2). These transporters expressed at a considerable level on day 21, and were maintained up to day 40. In the hiPSC-derived enterocytes of the present invention, the directional B-to-A transcellular transport of digoxin and prazosin was clearly observed, and the directional transport was inhibited by verapamil and elacridar. Digoxin is a selective substrate of P-gp, and verapamil potently inhibits P-gp with $K_i/IC_{50}$ values of 8.1 to 17.3 μM. On the other hand, prazosin is a good substrate of BCRP and P-gp, and elacridar inhibits both BCRP ($K_i/IC_{50}$ value: 0.31 μM) and P-gp ($K_i/IC_{50}$ value: 0.027 to 0.44 μM). Thus, the transport experiments performed by the present inventors indicated that the P-gp and BCRP in the hiPSC-derived enterocytes function to repress the absorptive A-to-B membrane permeation.

The transport activity of digoxin in the hiPSC-derived enterocytes was similar to that measured in the Caco-2 cells (Djuv and Nilsen, (2008) Phytother. Res. 22, 1623-1628), and a ratio of B-to-A/A-to-B transport activity of prazosin was also similar to that in the Caco-2 cells reported previously (Wright et al., (2011) Eur. J. Pharmacol. 672, 70-76). As for paracellular transport, transport clearance of mannitol across a Caco-2 cell monolayer was reported to be 3%/$cm^2$/hr (Cogburn JN et al., (1991) Pharm Res. 8, 210-216). In the hiPSC-derived enterocytes, the transport clearance of mannitol was estimated to be 8.6%/$cm^2$/hr. This is reasonable because the paracellular transport was more limited in the Caco-2 cells compared with intact intestinal epithelial cells due to more rigid tight junction of the Caco-2 cells. As for transcellular transport, propranolol was reported to show 41.9 x $10^{-6}$ cm/sec in the Caco-2 cells (Artursson, (1990) J. Pharm. Sci. 79, 476-482) (which corresponds to approximately 99.4 μE/120 min/well). This is comparable to the present result (48 μE/120 min/well). Taking all results into consideration, it was confirmed that the formation of static and active barriers by the monolayer of our iPSC-derived enterocytes was similar to barrier formation of the Caco-2 cells.

In summary, according to the results obtained by the present inventors, the CVM can be used for promoting the differentiation into enterocytes, and for maintaining the expression levels of transporters and CYP enzymes for approximately 3 weeks.

Another important finding is that the hiPSC-derived endoderm or intestinal precursor cells on day 10 or day 15 can be cryopreserved. When the cryopreserved cells are thawed and seeded onto a CVM, these cells form a monolayer, show membrane integrity within two weeks, and further mature into functional enterocytes. Cryopreserved day 10 intestinal precursor cells are able to differentiate into enterocytes that express transporters and metabolizing enzymes at levels similar to those derived from day 3 DE cells. Cryopreserved day 15 intestinal precursor cells also differentiate into enterocytes although the expression levels of the transporters and CYP enzymes are lower than those obtained from hiPSC-derived day 3 DE cells. The results obtained by the present inventors indicate that use of a cryopreserved day 10 or day 15 intestinal precursor cell stock provides rapid differentiation into enterocytes.

It is also indicated that the protocol of the present inventors is applicable to different hiPSC lines. The present inventors used two hiPSC lines, and found that enterocytes derived from either cell line exhibit similar levels of ABCB1 expression and CYP enzyme expression. In the ChiPS18 cell-derived enterocytes, however, higher ABCG2 and SLC15A1 expression was observed.

In conclusion, by using a CVM, functional enterocytes that exhibit directional transporter-mediated permeation of drugs, and show permeability similar to that of the Caco-2 cells were successfully produced. The hiPSC-derived enterocytes showed the metabolic activity of CYP3A4. In summary, the results obtained by the present inventors therefore indicate that the present hiPSC-derived enterocyte model would be useful for pharmacological studies on drug absorption, drug-drug interaction, and the like.

(C) Materials and Methods

(1) Human iPS Cell Lines

Two human iPS cell lines, ChiPS18 (Asplund et al., 2015) (TakaraBio) and RPChiPS771 (ReproCell), were used. Undifferentiated cells were maintained in AK02 StemFit medium (Ajinomoto) on a cell culture dish (Invitrogen) pre-coated with Synthemax II (Corning Cat No.3535). For methionine deprivation, ChiPS18 and RPChiPS771 cells were cultured in complete StemFit medium (Ajinomoto) or Met-deprived KA01 medium (Ajinomoto).

(2) Preparation of CVM Insert

A collagen xerogel membrane is manufactured by Kanto Chemical Co., Inc. (Tokyo, Japan). Briefly, a CVM was prepared by the following three processes as previously reported (Oshikata-Miyazaki and Takezawa, 2016, Cytotechnology 68, 1801-1811; Yamaguchi et al., 2013, Toxicol. Sci. 135, 347-355; Yamaguchi et al., 2016, J. Appl. Toxicol. 36, 1025-1037): A gelation step 1) of causing 0.2 mL of 0.25% type I collagen sol to form an opaque soft gel in a culture dish with a diameter of 35 mm; a vitrification step 2) of causing the gel to become a rigid material through sufficient drying; and a rehydration step 3) of converting the vitrified material into a thin transparent gel membrane with enhanced gel strength by moisture supplementation. Subsequently, a collagen xerogel membrane defined as a dried CVM not containing free water was prepared by vitrifying again the collagen vitrigel membrane on a separable sheet. The collagen xerogel membrane was pasted onto a bottom end of a plastic cylinder with an inner-outer diameter of 11 to 13 mm and a length of 15 mm, and two hangers were connected to an upper end of the cylinder. Thus, a collagen xerogel membrane chamber that can be easily converted into a CVM chamber by rehydration with a culture medium can be produced as previously reported (Oshikata-Miyazaki and Takezawa, 2016, Cytotechnology 68, 1801-1811; Yamaguchi et al., 2013, Toxicol. Sci. 135, 347-355; Yamaguchi et al., 2016, J. Appl. Toxicol. 36, 1025-1037).

(3) Differentiation of iPS Cells into Enterocytes Undifferentiated ChiPS18 or RPChiPS771 cells were first differentiated into the endoderm using M15 cells. Briefly, a plate having a diameter of 100 mm was precoated with mitomycin-treated frozen M15 feeder cells at a density of 5 x $10^6$ cells/dish. For endoderm differentiation, undifferentiated ChiPS18 or RPChiPS771 cells were plated onto the M15 cell-coated plate with a diameter of 100 mm at a density of 5 x $10^5$ cells/dish, and cultured in M1 medium, that is, an endoderm differentiation medium supplemented with 3 $\mu$M CHIR99021 (Wako, Tokyo), for 1 day, then the medium was changed to M1 medium (not containing CHIR99021), and the cells were cultured for another 2 days. The M1 medium contains DMEM (ThermoFisher), 4,500 mg/L glucose, NEAA (ThermoFisher), L-Gln (Nacalai Tesque), PS (Nacalai Tesque), 0.1mM $\beta$-ME (Sigma), serum free B27 supplement (#12587001, ThermoFisher), and 100 ng/mL recombinant human activin A (Cell Guidance Systems, Cambridge, UK). On day 3 (D3), ChiPS18 or RPChiPS771-derived endodermal cells were collected, and frozen at 2.0 x $10^6$ cells/mL in Bambanker hRM (NIPPON Genetics Co., Ltd., CS-07-001) and stocked in liquid nitrogen until further use.

For preparation of cryopreserved day 10 or day 15 intestinal precursor cells instead of a cryopreserved day 3 DE stock, the differentiation culture was continued on M15 cells, and the medium was changed to M2 medium on day 3. The M2 medium contains DMEM (Gibco, 11885-084, low glucose) supplemented with NEAA, L-Gln, PS, 0.1mM $\beta$-ME, D-Glucose 1mg/ml, 10% KnockOut (Registered trademark) Serum Replacement (Gibco, 10828028), 5$\mu$M 6-Bromoindirubin-3'-oxime (WAKO, 029-16241), and 10 $\mu$M DAPT (WAKO, 049-33583).

For intestine differentiation of cryopreserved cells, the day 3 DE cells were freeze thawed, and plated onto a rehydrated CVM 24-well insert (ad-MED Vitrigel ™ 2, KANTO KAGAKU, culture area: 0.33 $cm^2$/insert) at a concentration of 8 x $10^4$ cells/well. A volume of the medium was 200 $\mu$L for an upper layer and 500 $\mu$L for a lower layer of the insert. Media used for differentiation were M2 medium for days 3 to 15, and M3 medium for days 15 to 21, or days 15 to 40. The M3 medium contains Cellartis (Registered trademark) Hepatocyte Maintenance Medium (Takara Bio Y30051). The media in both the upper and lower layers were replaced every 2 days with fresh media and growth factors. For intestine differentiation using the day 10 or day 15 cryopreserved cell stock, cells were thawed and plated onto a CVM 24-well insert at a concentration of 1.6 x $10^5$ cells/well. For the day 10 cells, M2 medium was used until day 15, then the medium was changed to M3 medium, and the resultant cells were cultured up to day 40. For the day 15 cells, M3 medium was used to culture the cells up to day 40.

(4) Revised Protocol for Preparation of Cryopreserved Day 10 Intestinal Precursor Cells

For preparation of cryopreserved day 10 intestinal precursor cells, day 3 DE cells were plated onto a normal tissue culture plate (or CellSeed, CS3005) having a diameter of 100 mm and precoated with iMatrix-551 (FUJIFILM WAKO, 380-13041) at a density of 4 x $10^6$ cells/dish, and cultured in M2 medium until day 10 (D10). The medium was replaced every 2 days with fresh M2 medium. Thereafter, the cells were collected and frozen as a cryopreserved day 10 intestinal precursor cell stock.

For intestine differentiation, the day 10 intestinal precursor cell stock was freeze thawed, and plated onto a rehydrated vitrigel membrane (CVM) 24-well insert (ad-MED Vitrigel ™ 2, KANTO KAGAKU, culture area: 0.33 $cm^2$/insert) in M2 medium at a concentration of 1.6 x $10^5$ cells/well. Y-27632 (WAKO: 251-00514) was added to the medium upon plating for the first 2 days. The cells were cultured in M2 until day 15. A volume of the medium was 200 $\mu$L for an upper layer and 700 $\mu$L for a lower layer of the cell culture insert. The medium was changed to M3-1 or M3-2 for days 15 to 21, or up to day 30. The medium was replaced every 2 days with a fresh medium.

M3-1 medium: William's Medium E supplemented with L-glutamine, HCM SingleQuots (containing neither GA1000 nor human EGF) (Lonza, CC-4182), 1% PS (Nacalai), 10 ng/ml recombinant human HGF (PeproTech 100-39), 1 $\mu$M dexamethasone (Sigma-Aldrich, D8893), 1.4 $\mu$M BIO, and 1 $\mu$M calcitriol (Sigma-Aldrich, D1530).

M3-2 medium: The same components as M3-1 medium except that 0.5% dimethyl sulfoxide (DMSO; Sigma-Aldrich, D2650) is used instead of HGF.

(5) Culture of Caco-2 Cells

The Caco-2 cells were cultured in DMEM medium (low glucose) supplemented with 10% fetal bovine serum in a tissue culture dish having a diameter of 10 cm at 5.0 x $10^5$ cells/ml, and were passaged every 3 to 4 days at approximately 80% confluency. The Caco-2 cells were freshly freeze thawed, and passaged once before use. For measurement of membrane integrity, the Caco-2 cells were passaged onto a CVM 24-well insert at 1.0 x $10^4$ cells/well, and the culture was continued. The Caco-2 cells typically formed a monolayer after approximately 7 days of the culture.

(6) TEER Measurement

Membrane integrities of human iPSC-derived enterocytes and the Caco-2 cells cultured on a CVM were measured using TEER Measuring System (Kanto kagaku, Inc., Tokyo).

(7) Immunocytochemistry

Cells were fixed in 4% paraformaldehyde (Nacalai Tesque) in PBS, permeabilized with 0.1% Triton X-100 (Nacalai Tesque), and then blocked with 20% Blocking One (Nacalai Tesque, Japan) in PBST (0.1% Tween-20 in PBS). Antibodies were diluted in 20% Blocking One (Nacalai Tesque, Japan) in PBST (0.1% Tween-20 in PBS). Cells were counterstained with 6-diamidino-2-phenylindole (DAPI) (Roche Diagnostics, Switzerland).

The following antibodies were used: Anti-SOX17 (R&D systems, Minneapolis, MN), anti-CDX2 (BioGenex, San Ramon, CA), anti-ZOI (R40.76; Santa Cruz sc-33725), anti-VILLIN (BD Transduction Laboratories, San Diego), Alexa 568-conjugated and Alexa 488-conjugated antibodies

(Invitrogen) .

(8) Real Time PCR analysis

RNA was extracted from iPS cells using the RNeasy micro-kit or Qiaxol (Qiagen, Germany), and then treated with DNase (Qiagen). For a reverse transcription reaction, 2.5 $\mu$g of RNA was reverse-transcribed using PrimeScript ™ RT Master Mix (Takara, Japan). For real-time PCR analysis, mRNA expression was quantified with SyberGreen on a StepOne Plus (Applied Biosystems, Foster City, CA). The PCR conditions were as follows: a cycle of initial denaturation at 95°C for 30 sec, followed by denaturation at 95°C for 5 sec, and annealing and extension at 60°C for 30 sec were repeated 40 times. A target mRNA level was expressed as an arbitrary unit, and was determined using the $\Delta\Delta$ CT method. The details of primers are shown in Table 1 below. Besides, sequences of the primers are set forth in SEQ ID NOs: 1 to 18.

[Table 1]

| Gene name | Primer Sequence (F) | Primer Sequence (R) |
|---|---|---|
| *LGR5* | TAACTGGAACTGCAAACCTGGAGA | CTGATTGCAGACGGTTTGAGGA |
| *CDX2* | TCACTGGGCATTTCCGTGAG | GTGGATCGGCCAGATAACAAGA |
| *VIL1* | CGACTGCTACCTGCTGCTCTACAC | CGGCTTGATAAGCTGATGCTGTAA |
| *ABCB1* | GGAGCCTACTTGGTGGCACATAA | TGGCATAGTCAGGAGCAAATGAAC |
| *ABCG2* | GTTCCCAAATTGATGTTGTGACAGA | GTTCCCAAATTGATGTTGTGACAGA |
| *SLC15A1* | TCACCTGTGGCGAAGTGGTC | AGCAGCCATCCTGCCTGAA |
| *CYP2C9* | AACACTGCAGTTGACTTGTTTGGAG | GGTTTCTGCCAATCACACGTTC |
| *CYP2C19* | AATCACTGCAGCTGACTTACTTGG | CCGGTTTCTGCCAATGACAC |
| *CYP3A4* | GAAACACAGATCCCCCTGAA | CTGGTGTTCTCAGGCACAGA |

(9) Transcellular Transport Assay

For selectively evaluating the transporter activity of P-gp, time-dependent directional transport (B-to-A transport and A-to-B transport) of [$^3$H]-digoxin (0.1 μCi/ 3mL) across a cell monolayer was measured in the absence or presence of 100 μM verapamil. For evaluating BCRP and P-gp activities, time-dependent directional transport of [$^3$H]-prazosin (0.1 μCi/ 3mL) was measured in the absence or presence of 20 μM elacridar. For evaluating transcellular transport mediated by passive membrane permeation and paracellular transport across a cell monolayer, [$^3$H]-propranolol (0.1 μCi /3mL) and [$^3$H]-mannitol (0.1 μCi/ 3mL) were respectively tested.

In the transcellular transport assay, a hiPS-derived enterocyte culture medium was removed and replaced with a transport buffer (TB; 118 mM NaCl, 23.8 mM $Na_2CO_3$, 4.8 mM KCl, 1.0 mM $KH_2PO_4$, 1.2 mM $MgSO_4$, 12.5 mM HEPES, 5 mM glucose, and 1.5 mM $CaCl_2$, adjusted at pH7.4) at 37°C, and pre-incubation was performed for 10 min. Assays were started by replacing the TB with TB containing a substrate +/- inhibitor. Volumes of the TB added were 200 μl to an upper layer and 500 μl to a lower layer of a CVM insert. Then, 30, 60 and 120 min after starting drug incubation, aliquots of the medium at the opposite compartment of initial drug application were sampled and replaced with the same volume of fresh TB (substrate +/inhibitor). For the measurement of the A-to-B transport, 100 μl of TB was sampled from a lower compartment, and for the measurement of the B-to-A transport, 50 μl of TB was sampled from an upper compartment. Then, the thus obtained samples were mixed with CLEAR-SOL I (Nacalai Tesque), and their radioactivity was quantified with a liquid scintillation counter (PerkinElmer).

Alternatively, rhodamine 123 (10 μM; Dojindo, R233) was used, as a substrate to evaluate the transporter activity of P-gp, in the absence or presence of cyclosporine A (10 μM) (Wako CAS RN 59865-13-3), that is, an inhibitor of P-gp. A flux of rhodamine 123 was determined with a luminometer (GloMax Microplate Luminometer, Promega). Differentiated cells showing TEER > 30 Ω.cm$^2$ were used. The hiPS-derived enterocyte culture medium was removed, then washed and replaced with 5% fetal bovine serum (FBS) supplemented with Hanks' Balanced Salt Solution (HBSS). Assays were started by replacing the FBS-containing HBSS buffer with a substrate (+/- cyclosporine), and the resultant was incubated at 37°C for 2 hours. An apparent membrane permeability coefficient (Papp) was calculated as follows: Papp = dQ/dt x 1/ACo

When dQ/dt is an amount of the compound permeated per unit time, A is a surface area of a cell culture insert membrane (0.33 cm$^2$), and Co is an initial compound concentration in a donor chamber. An efflux ratio of rhodamine123 was calculated as follows:

$$\text{Flux ratio} = P_{\text{app, basolateral to apical}} / P_{\text{app, apical to basolateral}}$$

(10) CYP Metabolism Assay

CYP3A4 activity in hiPS-derived enterocytes on day 21 of differentiation was evaluated. A hiPS-derived enterocyte culture medium (M3) was removed and replaced with a transport buffer (TB) at 37°C, and pre-incubation was performed for 10 min. Assays were started by adding TB containing midazolam to both an upper compartment (200 μl) and a lower compartment (500 μl) . After 120 min incubation, all the incubation media were collected from both the upper and lower compartments, and the thus obtained samples were kept at -80°C until performing LC-MS/MS analysis of 1'-OH midazolam, that is, a metabolite of midazolam. A protein amount per well was quantified using Pierce BCA protein assay kit (ThermoFisher, Rockford, IL) according to the manufacturer's instructions. Metabolic clearance was expressed as a value normalized to a cellular protein amount.

(11) Statistics

Data is expressed as a mean ± S.D. (n = 3). Differences among groups are analyzed by Student's t-tests or one-way ANOVA with the post-hoc Dunnett's test. Respective statistical analysis and P-values are noted in the description of each drawing. *p < 0.05 or **p < 0.01, obtained by Student's t-test; and §P < 0.05 or §§P < 0.01, obtained by one-way ANOVA with the post-hoc Dunnett's test are considered to be significant.

[Reference]

Ameri, J., Stahlberg, A., Pedersen, J., Johansson, J. K., Johannesson, M. M., Artner, I. and Semb, H. (2010). FGF2 specifies hESC-derived definitive endoderm into foregut/midgut cell lineages in a concentration-dependent manner. Stem Cells 28, 45-56.
Artursson, P. (1990). Epithelial Transport Of Drugs In Cell Culture. I: A Model For Studying The Passive Diffusion Of Drugs Over Intestinal Absorbtive (Caco-2) Cells. J. Pharm. Sci. 79, 476-482.
Asplund, A., Pradip, A., van Giezen, M., Aspegren, A., Choukair, H., Rehnstrom, M., Jacobsson, S., Ghosheh, N., El Hajjam, D., Holmgren, S., et al. (2015). One Standardized Differentiation Procedure Robustly Generates Homogenous Hepatocyte Cultures Displaying Metabolic Diversity from a Large Panel of Human Pluripotent Stem Cells. Stem Cell Rev. Reports.
Barker, N., van Es, J. H., Kuipers, J., Kujala, P., van den Born, M., Cozijnsen, M., Haegebarth, A., Korving, J., Begthel, H., Peters, P. J., et al. (2007). Identification of stem cells in small intestine and colon by marker gene Lgr5. Nature 449, 1003-7.
Buczacki, S. J. a, Zecchini, H. I., Nicholson, A. M., Russell, R., Vermeulen, L., Kemp, R. and Winton, D. J. (2013). Intestinal label-retaining cells are secretory precursors expressing Lgr5. Nature 495, 65-9. Chiba, S. (2006). Notch signaling in stem cell systems. Stem Cells 24, 2437-47.
Cogburn, J. N., Donovan, M. G. and Schasteen, C. S. (1991). A model of human small intestinal absorptive cells. 1. Transport barrier. Pharm. Res. 8, 210-6.
Djuv, A. and Nilsen, O. G. (2008). Review of pharmacological effects of Glycyrrhiza radix and its bioactive compounds. Phytother. Res. 22, 1623-1628.
Estudante, M., Morais, J. G., Soveral, G. and Benet, L. Z. (2013). Intestinal drug transporters: An overview. Adv. Drug Deliv. Rev. 65, 1340-1356.
Fre, S., Huyghe, M., Mourikis, P., Robine, S., Louvard, D. and Artavanis-Tsakonas, S. (2005). Notch signals control the fate of immature progenitor cells in the intestine. Nature 435, 964-8.
Fre, S., Pallavi, S. K., Huyghe, M., Lae, M., Janssen, K.-P., Robine, S., Artavanis-Tsakonas, S. and Louvard, D. (2009). Notch and Wnt signals cooperatively control cell proliferation and tumorigenesis in the intestine. Proc. Natl. Acad. Sci. U. S. A. 106, 6309-14.
Gerbe, F., van Es, J. H., Makrini, L., Brulin, B., Mellitzer, G., Robine, S., Romagnolo, B., Shroyer, N. F., Bourgaux, J.-F., Pignodel, C., et al. (2011). Distinct ATOH1 and Neurog3 requirements define tuft cells as a new secretory cell type in the intestinal epithelium. J. Cell Biol. 192, 767-80.
Gregorieff, A., Liu, Y., Inanlou, M. R., Khomchuk, Y. and Wrana, J. L. (2015). Yap-dependent reprogramming of Lgr5+ stem cells drives intestinal regeneration and cancer. Nature.
Hansson, M., Olesen, D. R., Peterslund, J. M. L., Engberg, N., Kahn, M., Winzi, M., Klein, T., Maddox-Hyttel, P. and Serup, P. (2009). A late requirement for Wnt and FGF signaling during activin-induced formation of foregut endoderm from mouse embryonic stem cells. Dev. Biol. 330, 286-304.
Hubatsch, I., Ragnarsson, E. G. E. and Artursson, P. (2007). Determination of drug permeability and prediction of drug absorption in Caco-2 monolayers. Nat. Protoc. 2, 2111-2119.
Iwao, T., Kodama, N., Kondo, Y., Kabeya, T., Nakamura, K., Horikawa, T., Niwa, T., Kurose, K. and Matsunaga, T. (2015). Generation of Enterocyte-Like Cells with Pharmacokinetic Functions from Human Induced Pluripotent Stem Cells Using Small- Molecule Compounds. Drug Metab. Dispos. 43, 603-610.
Kodama, N., Iwao, T., Katano, T., Ohta, K., Yuasa, H. and Matsunaga, T. (2016). Characteristic Analysis of Intestinal Transport in Enterocyte-Like Cells Differentiated from Human Induced Pluripotent Stem Cells. Drug Metab. Dispos. 44, 0-0.
Lancaster, M. a. and Knoblich, J. a. (2014). Organogenesis in a dish: modeling development and disease using organoid technologies. Science 345, 1247125. Nakamura, T., Tsuchiya, K. and Watanabe, M. (2007). Crosstalk between Wnt and Notch signaling in intestinal epithelial cell fate decision. J. Gastroenterol. 42, 705-10.
Negoro, R., Takayama, K., Nagamoto, Y., Sakurai, F., Tachibana, M. and Mizuguchi, H. (2016). Modeling of drug-mediated CYP3A4 induction by using human iPS cell-derived enterocyte-like cells. Biochem. Biophys. Res. Commun. 472, 631-6.

Ogaki, S., Shiraki, N., Kume, K. and Kume, S. (2013). Wnt and Notch signals guide embryonic stem cell differentiation into the intestinal lineages. Stem Cells 31, 1086-96.

Ogaki, S., Morooka, M., Otera, K. and Kume, S. (2015). A cost-effective system for differentiation of intestinal epithelium from human induced pluripotent stem cells. Sci. Rep. 5, 17297.

Oshikata-Miyazaki, A. and Takezawa, T. (2016). Development of an oxygenation culture method for activating the liver-specific functions of HepG2 cells utilizing a collagen vitrigel membrane chamber. Cytotechnology 68, 1801-1811.

Sato, T. and Clevers, H. (2013). Growing Self-Organized Mini-Guts from a Single Intestinal Stem Cell: Mechanisms and Applications. Science 340, 190-194.

Sato, T., Vries, R. G., Snippert, H. J., van de Wetering, M., Barker, N., Stange, D. E., van Es, J. H., Abo, A., Kujala, P., Peters, P. J., et al. (2009). Single Lgr5 stem cells build crypt-villus structures in vitro without a mesenchymal niche. Nature 459, 262-5.

Sato, T., Stange, D. E., Ferrante, M., Vries, R. G. J., Van Es, J. H., Van Den Brink, S., Van Houdt, W. J., Pronk, A., Van Gorp, J., Siersema, P. D., et al. (2011). Long-term expansion of epithelial organoids from human colon, adenoma, adenocarcinoma, and Barrett's epithelium. Gastroenterology 141, 1762-1772.

Spence, J. R., Mayhew, C. N., Rankin, S. a, Kuhar, M. F., Vallance, J. E., Tolle, K., Hoskins, E. E., Kalinichenko, V. V, Wells, S. I., Zorn, A. M., et al. (2011). Directed differentiation of human pluripotent stem cells into intestinal tissue in vitro. Nature 470, 105-9.

Sun, D., Lennernas, H., Welage, L. S., Barnett, J. L., Landowski, C. P., Foster, D., Fleisher, D., Lee, K. D. and Amidon, G. L. (2002). Comparison of human duodenum and Caco-2 gene expression profiles for 12,000 gene sequences tags and correlation with permeability of 26 drugs. Pharm. Res. 19, 4-6.

Takahashi, K., Tanabe, K., Ohnuki, M., Narita, M., Ichisaka, T., Tomoda, K. and Yamanaka, S. (2007). Induction of pluripotent stem cells from adult human fibroblasts by defined factors. Cell 131, 861-72.

Takano, J., Maeda, K., Bolger, M. B. and Sugiyama, Y. (2016). The prediction of the relative importance of CYP3A/P-glycoprotein to the nonlinear intestinal absorption of drugs by advanced compartmental absorption and transit model. Drug Metab. Dispos. 44, 1808-1818.

Watson, C. L., Mahe, M. M., Munera, J., Howell, J. C., Sundaram, N., Poling, H. M., Schweitzer, J. I., Vallance, J. E., Mayhew, C. N., Sun, Y., et al. (2014). An in vivo model of human small intestine using pluripotent stem cells. Nat. Med. 20,.

Wright, J. A., Haslam, I. S., Coleman, T. and Simmons, N. L. (2011). Breast cancer resistance protein BCRP (ABCG2)-mediated transepithelial nitrofurantoin secretion and its regulation in human intestinal epithelial (Caco-2) layers. Eur. J. Pharmacol. 672, 70-76.

Yamaguchi, H., Kojima, H. and Takezawa, T. (2013). Vitrigel-eye irritancy test method using HCE-T cells. Toxicol. Sci. 135, 347-355.

Yamaguchi, H., Kojima, H. and Takezawa, T. (2016). Predictive performance of the Vitrigel-eye irritancy test method using 118 chemicals. J. Appl. Toxicol. 36, 1025-1037.

All the publications, patents and patent applications cited herein are incorporated in their entirety by reference into the present specification.

Industrial Applicability

The present invention relates to enterocytes, and hence is applicable in industrial fields using the cells.

## Claims

1. An enterocyte differentiation medium comprising: a GSK3 inhibitor; and at least one selected from the group consisting of an activator of a hepatocyte growth factor receptor, an adrenal cortex hormone, calcitriol, and dimethyl sulfoxide.

2. The enterocyte differentiation medium according to claim 1, wherein the GSK3 inhibitor is BIO.

3. The enterocyte differentiation medium according to claim 1 or 2, wherein the activator of a hepatocyte growth factor receptor is HGF.

4. The enterocyte differentiation medium according to any one of claims 1 to 3, wherein the adrenal cortex hormone is dexamethasone.

**5.** A method for producing enterocytes, comprising the following steps (1) to (3):

(1) culturing pluripotent stem cells in a medium containing an activator of activin receptor-like kinase-4,7;
(2) culturing the cells resulting from the step (1) in a medium containing a GSK3 inhibitor and a γ-secretase inhibitor; and
(3) culturing the cells resulting from the step (2) on a high density collagen gel membrane in the enterocyte differentiation medium according to any one of claims 1 to 4, to obtain enterocytes.

**6.** The method for producing enterocytes according to claim 5, wherein the cells resulting from the step (1) are cultured on a high density collagen gel membrane in the step (2).

**7.** The method for producing enterocytes according to claim 5 or 6, wherein the pluripotent stem cells are cultured on M15 cells in the step (1).

**8.** The method for producing enterocytes according to any one of claims 5 to 7, wherein the activator of activin receptor-like kinase-4,7 is activin A in the step (1) .

**9.** The method for producing enterocytes according to any one of claims 5 to 8, wherein the GSK3 inhibitor is BIO, and the γ-secretase inhibitor is DAPT in the step (2) .

**10.** Enterocytes produced by the method according to any one of claims 5 to 9.

Figure 1

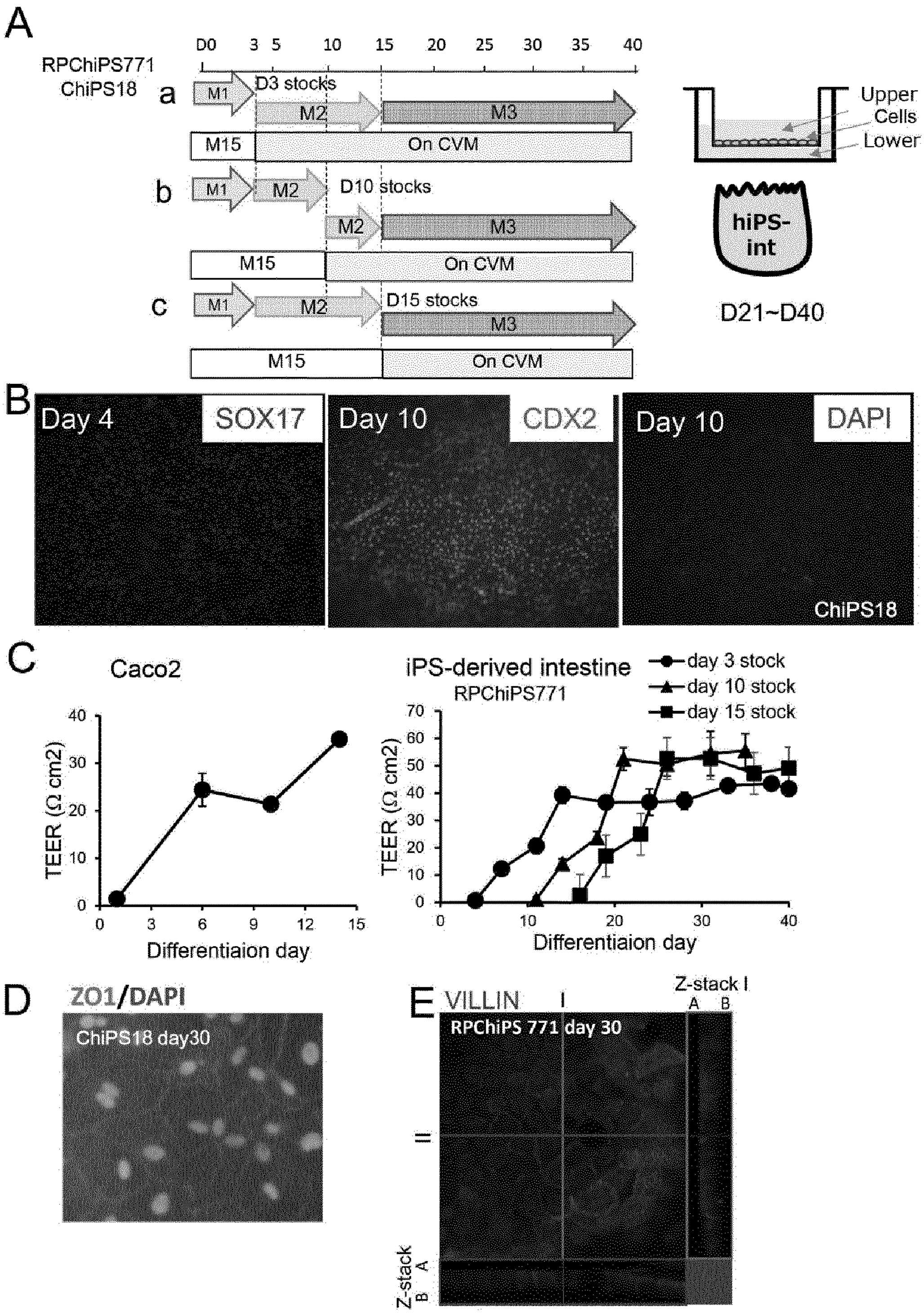
A
RPChiPS771
ChiPS18
D0 3 5 10 15 20 25 30 35 40
a
M1
D3 stocks
M2
M3
M15
On CVM
b
M1
M2
D10 stocks
M2
M3
M15
On CVM
c
M1
M2
D15 stocks
M3
M15
On CVM
Upper
Cells
Lower
hiPS-int
D21~D40
B
Day 4
SOX17
Day 10
CDX2
Day 10
DAPI
ChiPS18
C
Caco2
TEER (Ω cm2)
Differentiaion day
iPS-derived intestine
RPChiPS771
day 3 stock
day 10 stock
day 15 stock
TEER (Ω cm2)
Differentiaion day
D
ZO1/DAPI
ChiPS18 day30
E
VILLIN
Z-stack I
A B
RPChiPS 771 day 30
Z-stack
A
B

Figure 2

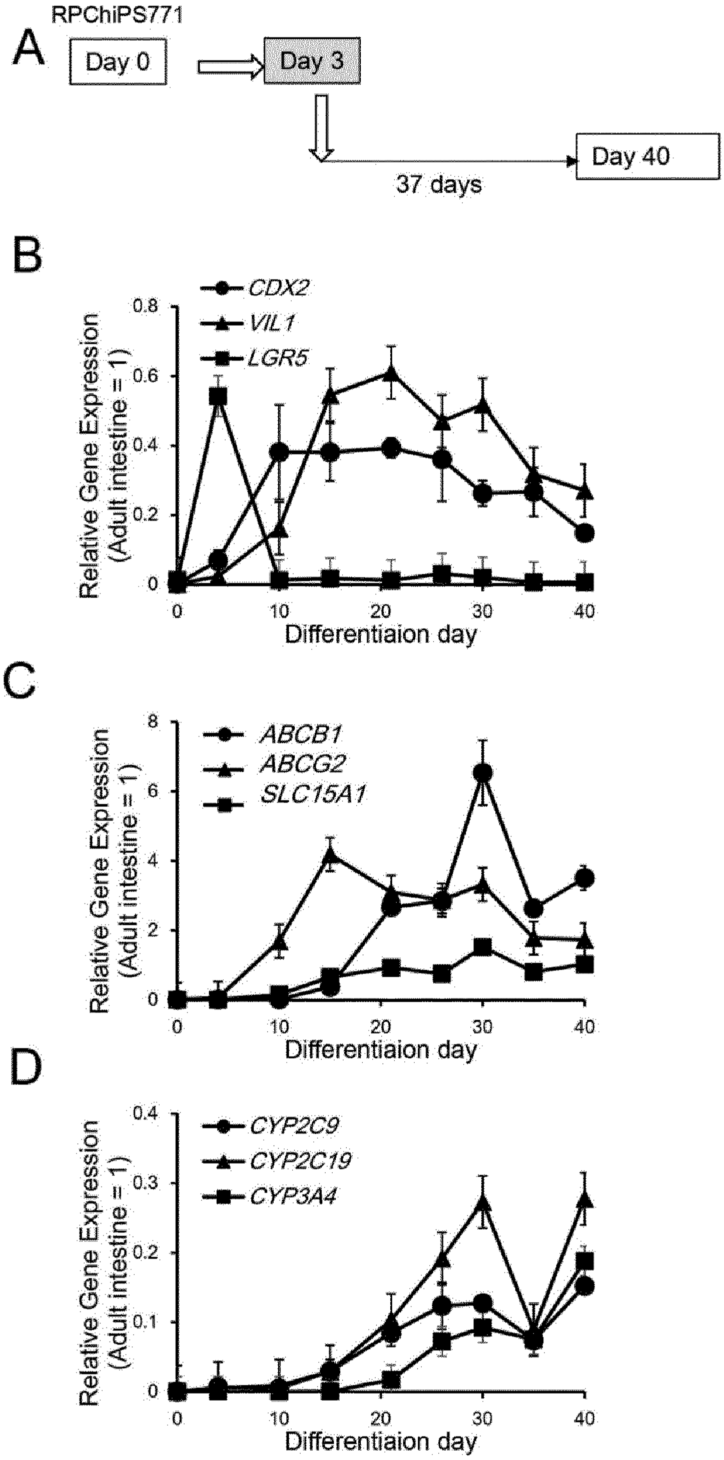
A
RPChiPS771
Day 0
Day 3
Day 40
37 days
B
CDX2
VIL1
LGR5
Relative Gene Expression
(Adult intestine = 1)
0.8
0.6
0.4
0.2
0
0
10
20
30
40
Differentiaion day
C
ABCB1
ABCG2
SLC15A1
Relative Gene Expression
(Adult intestine = 1)
8
6
4
2
0
0
10
20
30
40
Differentiaion day
D
CYP2C9
CYP2C19
CYP3A4
Relative Gene Expression
(Adult intestine = 1)
0.4
0.3
0.2
0.1
0
0
10
20
30
40
Differentiaion day

Figure 3

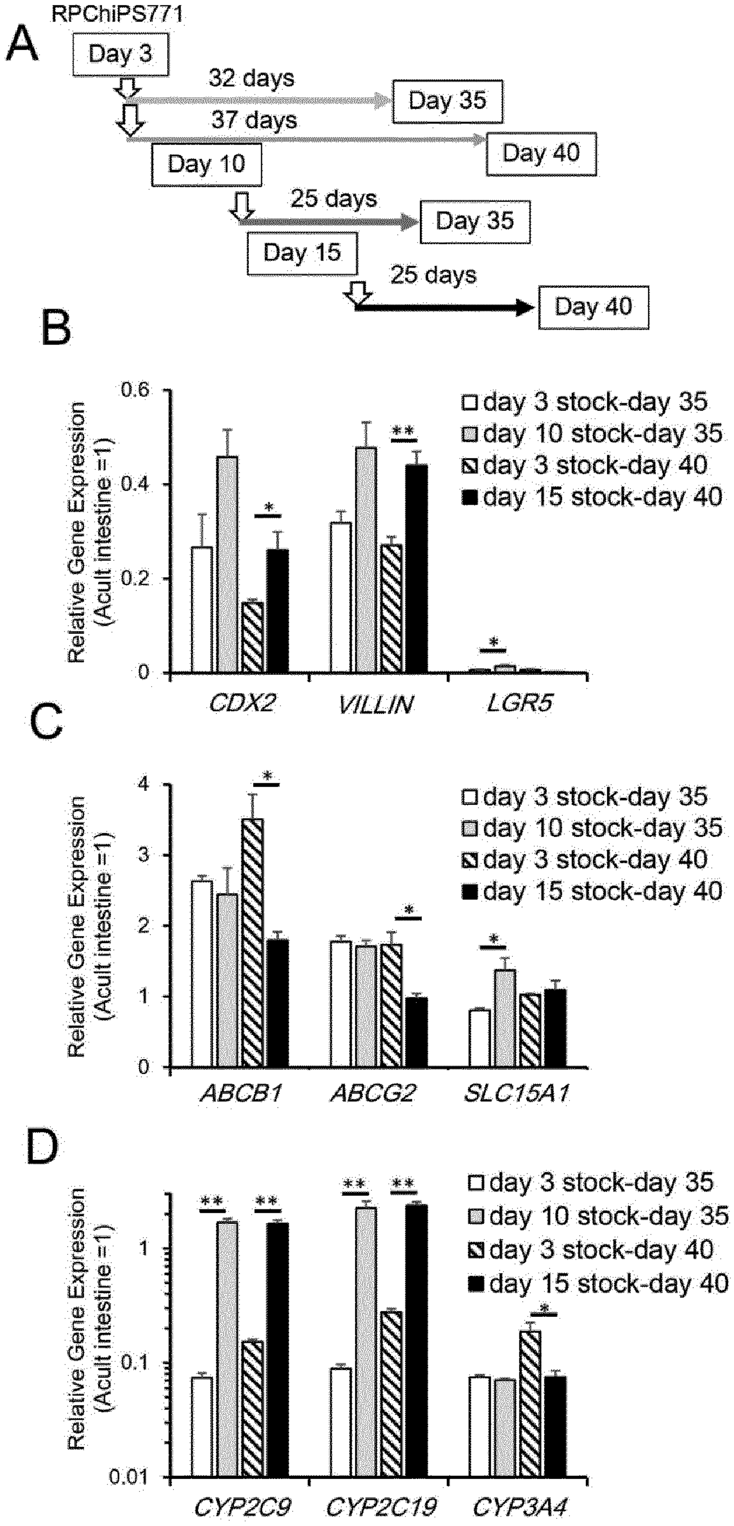
A
RPChiPS771
Day 3
32 days
Day 35
37 days
Day 40
Day 10
25 days
Day 35
Day 15
25 days
Day 40
B
Relative Gene Expression (Acult intestine =1)
0.6
0.4
0.2
0
day 3 stock-day 35
day 10 stock-day 35
day 3 stock-day 40
day 15 stock-day 40
CDX2
VILLIN
LGR5
C
Relative Gene Expression (Acult intestine =1)
4
3
2
1
0
day 3 stock-day 35
day 10 stock-day 35
day 3 stock-day 40
day 15 stock-day 40
ABCB1
ABCG2
SLC15A1
D
Relative Gene Expression (Acult intestine =1)
1
0.1
0.01
day 3 stock-day 35
day 10 stock-day 35
day 3 stock-day 40
day 15 stock-day 40
CYP2C9
CYP2C19
CYP3A4

Figure 4

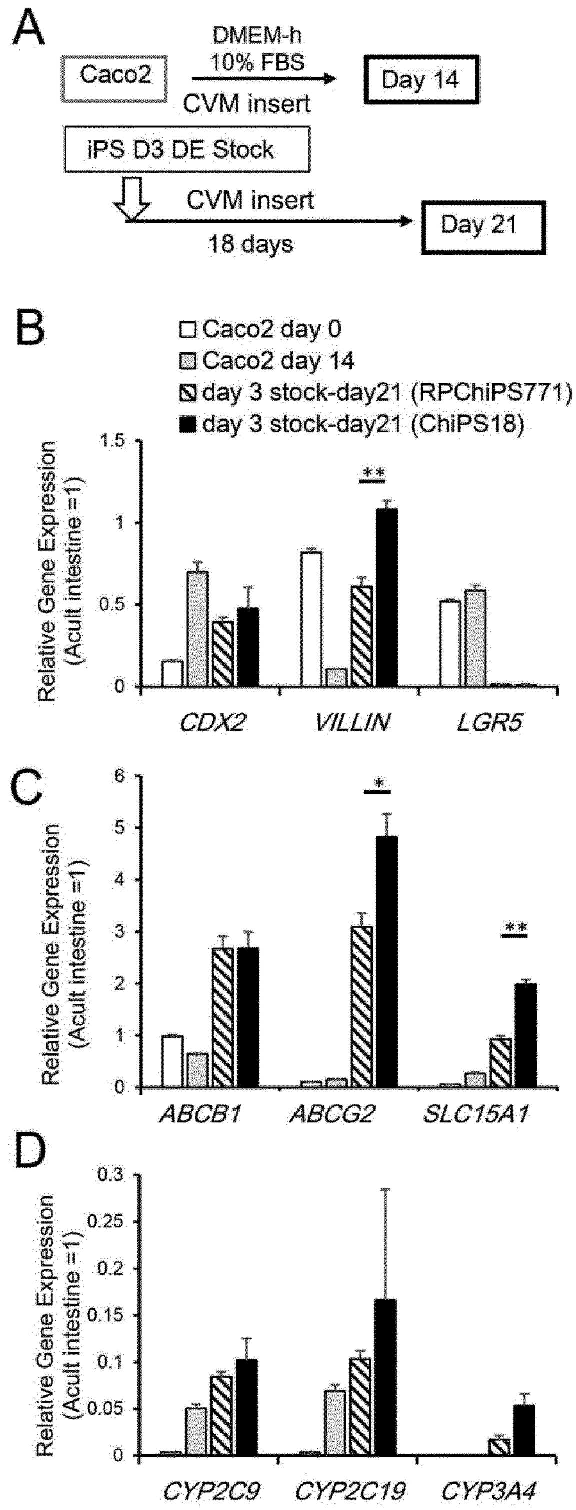
A
Caco2
DMEM-h
10% FBS
CVM insert
Day 14
iPS D3 DE Stock
CVM insert
18 days
Day 21
B
Caco2 day 0
Caco2 day 14
day 3 stock-day21 (RPChiPS771)
day 3 stock-day21 (ChiPS18)
Relative Gene Expression
(Acult intestine =1)
CDX2
VILLIN
LGR5
C
ABCB1
ABCG2
SLC15A1
D
CYP2C9
CYP2C19
CYP3A4

Figure 5

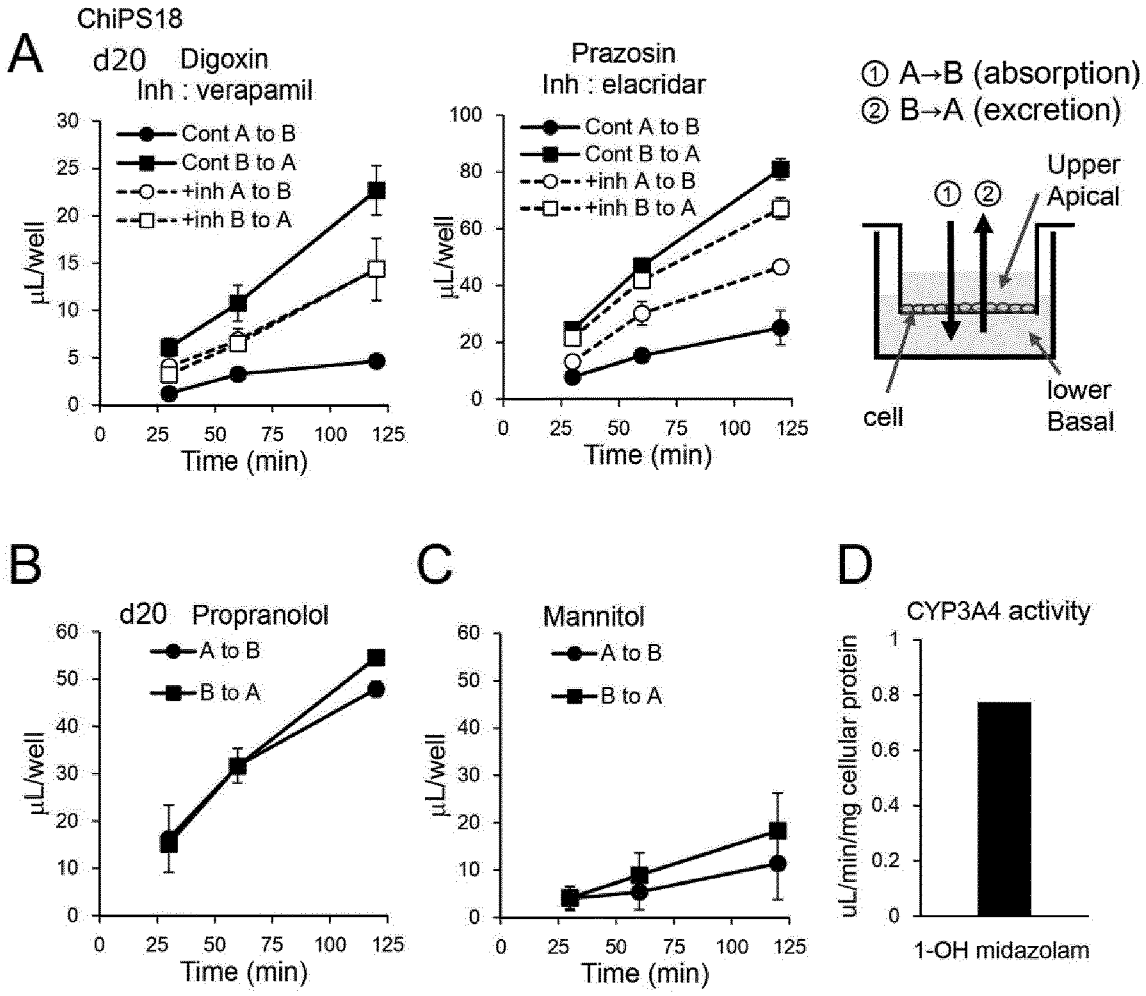
A
ChiPS18
d20 Digoxin
Inh : verapamil
Cont A to B
Cont B to A
+inh A to B
+inh B to A
μL/well
Time (min)
Prazosin
Inh : elacridar
Cont A to B
Cont B to A
+inh A to B
+inh B to A
μL/well
Time (min)
① A→B (absorption)
② B→A (excretion)
① ②
Upper
Apical
cell
lower
Basal
B
d20 Propranolol
A to B
B to A
μL/well
Time (min)
C
Mannitol
A to B
B to A
μL/well
Time (min)
D
CYP3A4 activity
uL/min/mg cellular protein
1-OH midazolam

Figure 6

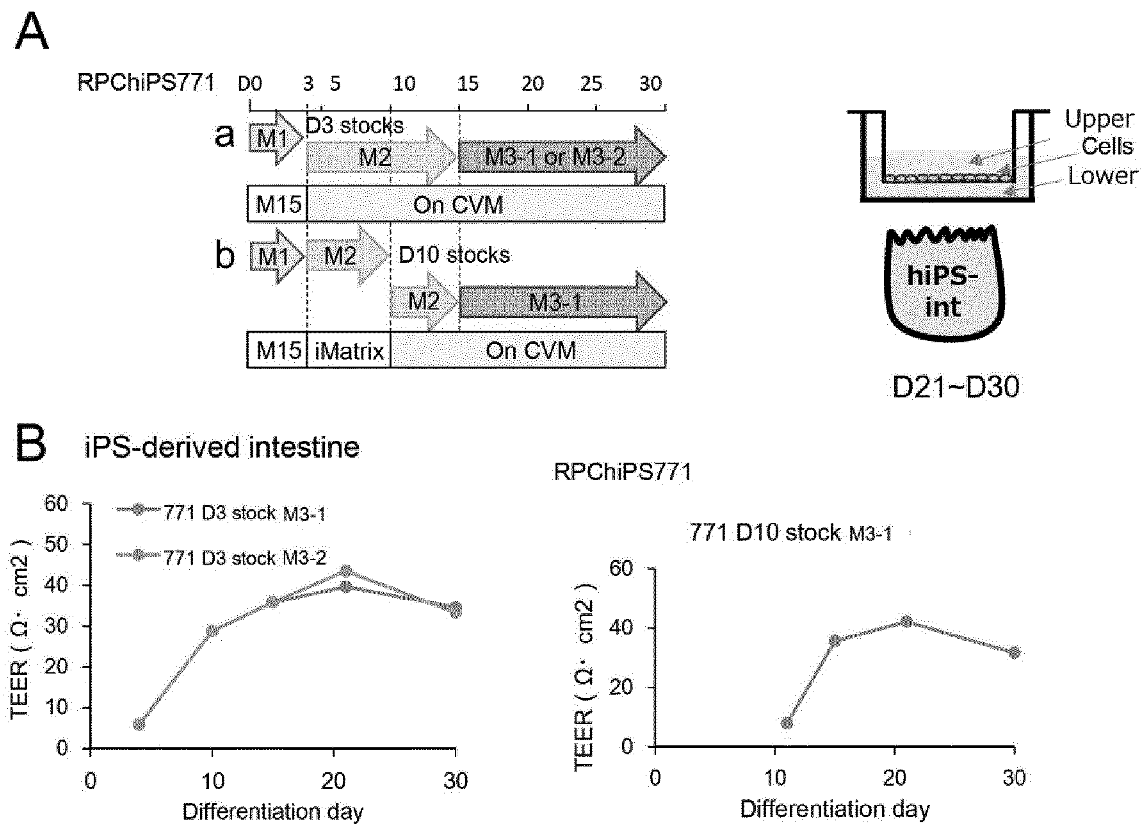
A
RPChiPS771
D0
3
5
10
15
20
25
30
a
M1
D3 stocks
M2
M3-1 or M3-2
M15
On CVM
b
M1
M2
D10 stocks
M2
M3-1
M15
iMatrix
On CVM
Upper
Cells
Lower
hiPS-int
D21~D30
B
iPS-derived intestine
771 D3 stock M3-1
771 D3 stock M3-2
TEER ( Ω· cm2 )
Differentiation day
RPChiPS771
771 D10 stock M3-1
TEER ( Ω· cm2 )
Differentiation day

Figure 7

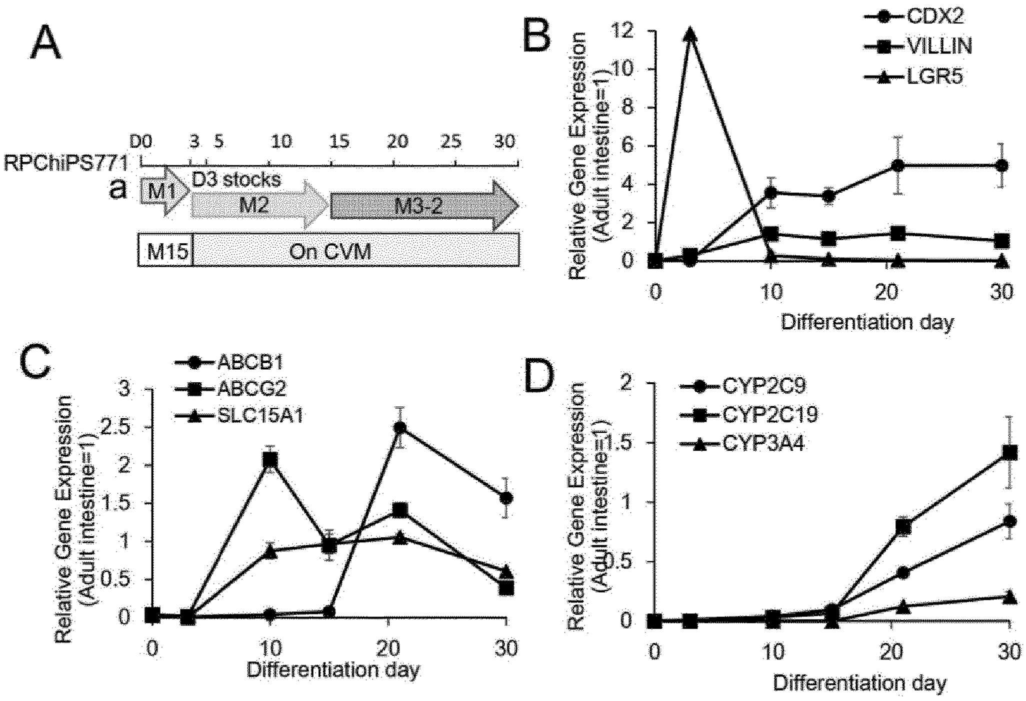
A
D0 3 5 10 15 20 25 30
RPChiPS771
a
M1
D3 stocks
M2
M3-2
M15
On CVM
B
CDX2
VILLIN
LGR5
Relative Gene Expression (Adult intestine=1)
Differentiation day
C
ABCB1
ABCG2
SLC15A1
Relative Gene Expression (Adult intestine=1)
Differentiation day
D
CYP2C9
CYP2C19
CYP3A4
Relative Gene Expression (Adult intestine=1)
Differentiation day

Figure 8

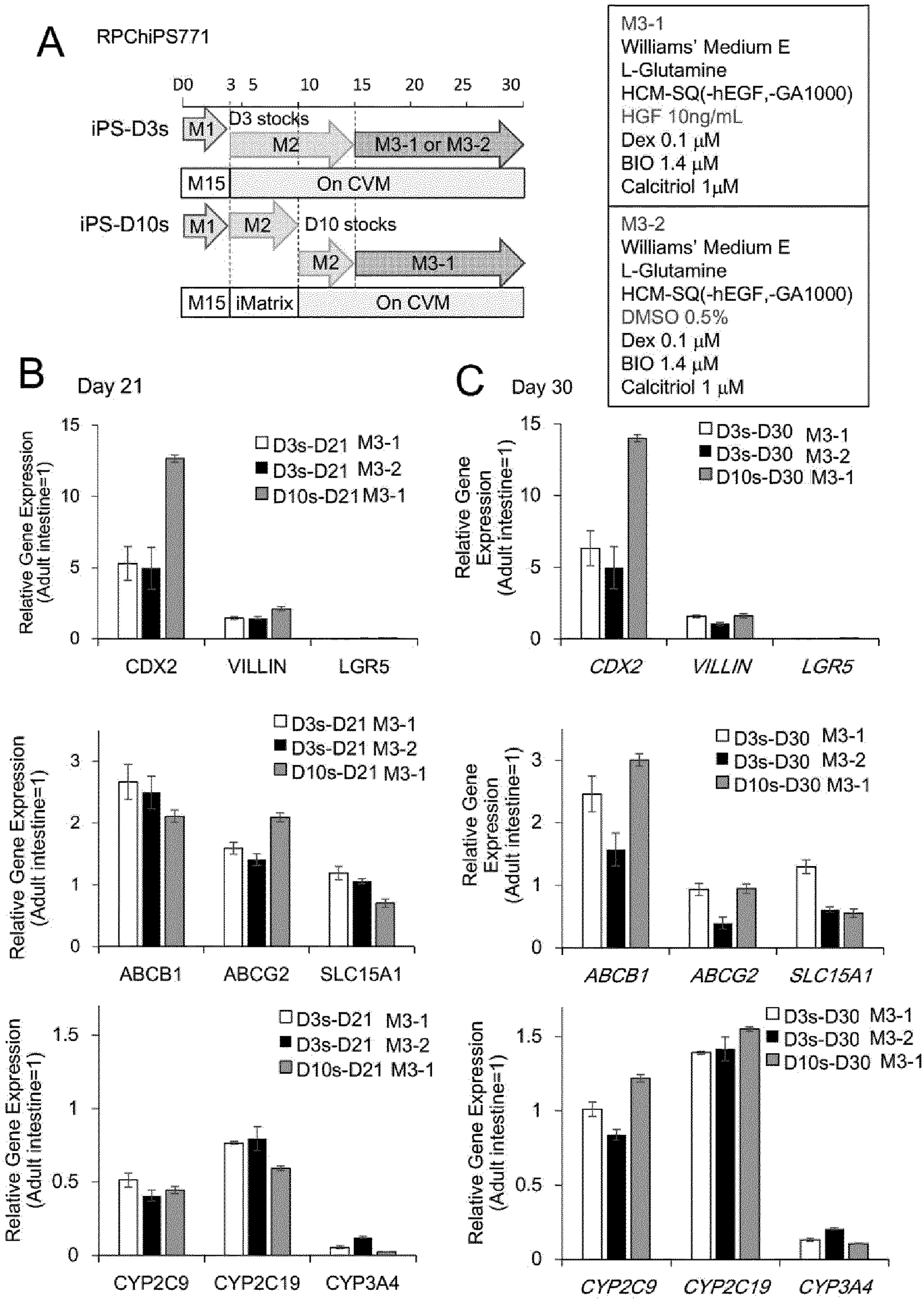
A
RPChiPS771
D0 3 5 10 15 20 25 30
iPS-D3s
M1
D3 stocks
M2
M3-1 or M3-2
M15
On CVM
iPS-D10s
M1
M2
D10 stocks
M2
M3-1
M15
iMatrix
On CVM
M3-1
Williams' Medium E
L-Glutamine
HCM-SQ(-hEGF,-GA1000)
HGF 10ng/mL
Dex 0.1 μM
BIO 1.4 μM
Calcitriol 1μM
M3-2
Williams' Medium E
L-Glutamine
HCM-SQ(-hEGF,-GA1000)
DMSO 0.5%
Dex 0.1 μM
BIO 1.4 μM
Calcitriol 1 μM
B
Day 21
Relative Gene Expression (Adult intestine=1)
D3s-D21 M3-1
D3s-D21 M3-2
D10s-D21 M3-1
CDX2
VILLIN
LGR5
ABCB1
ABCG2
SLC15A1
CYP2C9
CYP2C19
CYP3A4
C
Day 30
Relative Gene Expression (Adult intestine=1)
D3s-D30 M3-1
D3s-D30 M3-2
D10s-D30 M3-1
CDX2
VILLIN
LGR5
ABCB1
ABCG2
SLC15A1
CYP2C9
CYP2C19
CYP3A4

Figure 9

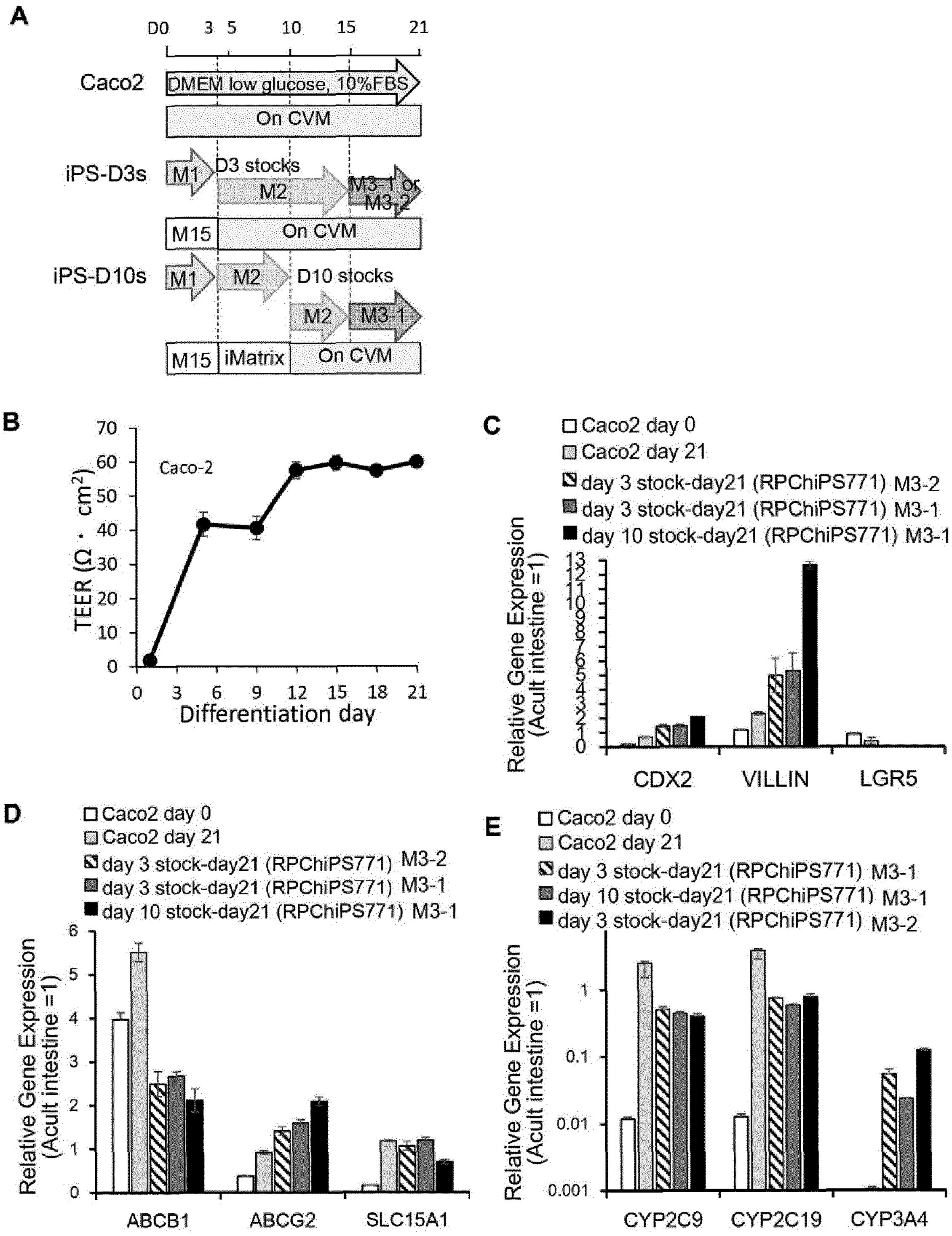
A
D0 3 5 10 15 21
Caco2
DMEM low glucose, 10%FBS
On CVM
iPS-D3s
M1
D3 stocks
M2
M3-1 or M3-2
M15
On CVM
iPS-D10s
M1
M2
D10 stocks
M2
M3-1
M15
iMatrix
On CVM
B
Caco-2
TEER (Ω · cm2)
Differentiation day
C
Caco2 day 0
Caco2 day 21
day 3 stock-day21 (RPChiPS771) M3-2
day 3 stock-day21 (RPChiPS771) M3-1
day 10 stock-day21 (RPChiPS771) M3-1
Relative Gene Expression (Acult intestine =1)
CDX2
VILLIN
LGR5
D
Caco2 day 0
Caco2 day 21
day 3 stock-day21 (RPChiPS771) M3-2
day 3 stock-day21 (RPChiPS771) M3-1
day 10 stock-day21 (RPChiPS771) M3-1
Relative Gene Expression (Acult intestine =1)
ABCB1
ABCG2
SLC15A1
E
Caco2 day 0
Caco2 day 21
day 3 stock-day21 (RPChiPS771) M3-1
day 10 stock-day21 (RPChiPS771) M3-1
day 3 stock-day21 (RPChiPS771) M3-2
Relative Gene Expression (Acult intestine =1)
CYP2C9
CYP2C19
CYP3A4

Figure 10

A On Day 21 RPChiPS771

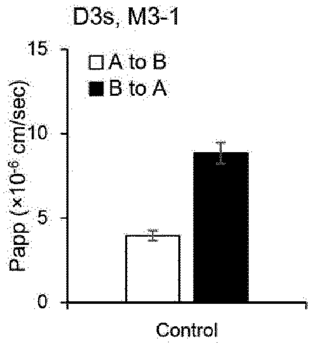


| | A to B | B to A | Flux ratio |
|---|---|---|---|
| Control | 3.95 | 8.8 | 2.24 |

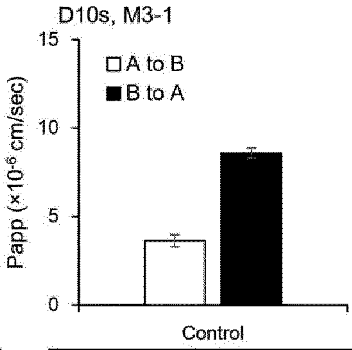


| | A to B | B to A | Flux ratio |
|---|---|---|---|
| Control | 3.63 | 8.6 | 2.37 |

On Day 30

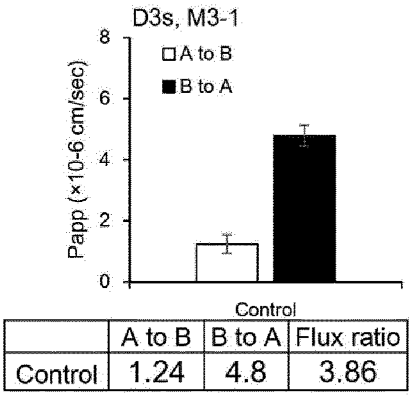


| | A to B | B to A | Flux ratio |
|---|---|---|---|
| Control | 1.24 | 4.8 | 3.86 |

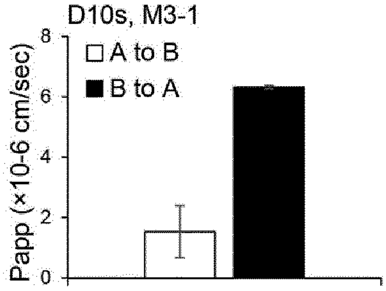


| | A to B | B to A | Flux ratio |
|---|---|---|---|
| Control | 1.53 | 6.3 | 4.13 |

B On Day 26

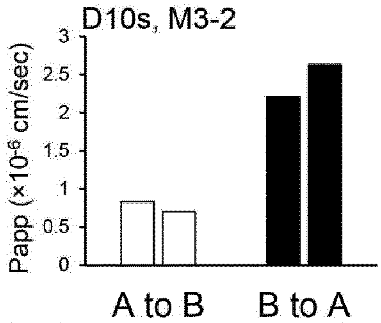


| | A to B | B to A | Flux ratio |
|---|---|---|---|
| #1 | 0.83 | 2.2 | 2.65 |
| #2 | 0.70 | 2.6 | |

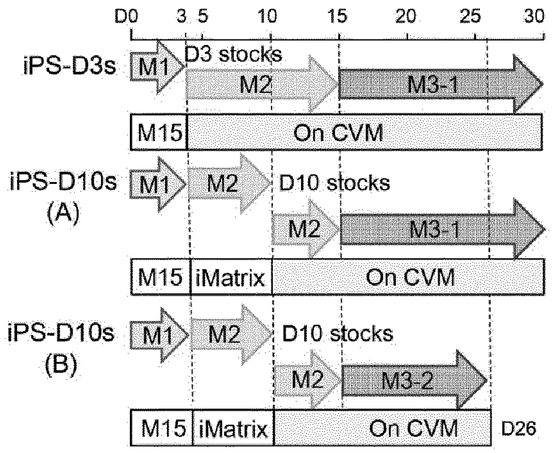

Figure 11

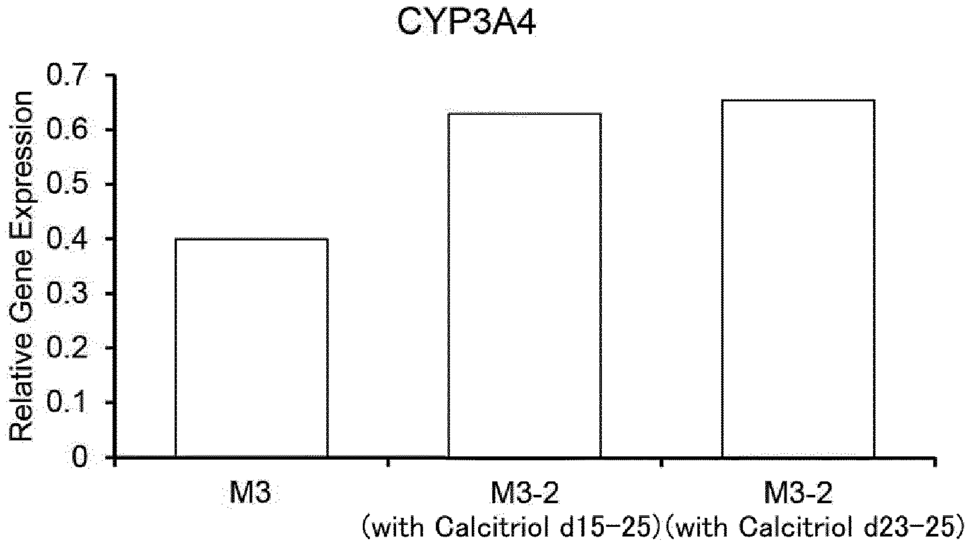


Figure 12

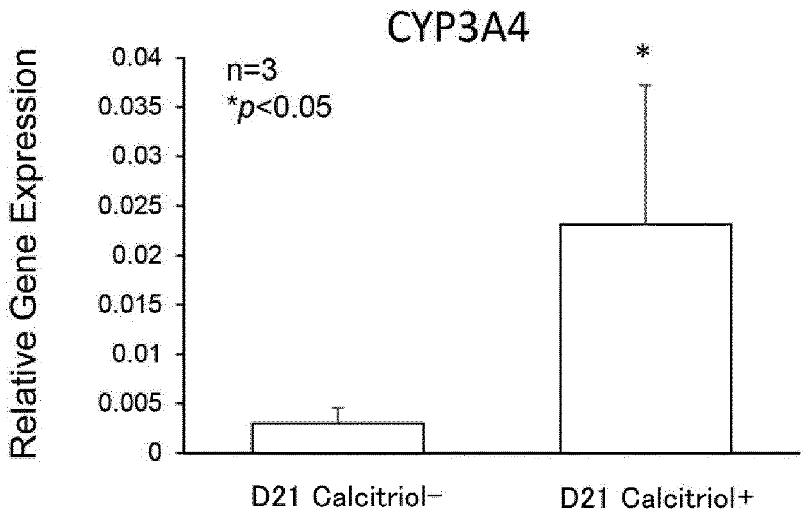

## INTERNATIONAL SEARCH REPORT

International application No. PCT/JP2019/035358

**A. CLASSIFICATION OF SUBJECT MATTER**

Int.Cl. C12N5/071(2010.01)i, C07K14/78(2006.01)n, C12N1/00(2006.01)n, C12N5/074(2010.01)n

According to International Patent Classification (IPC) or to both national classification and IPC

**B. FIELDS SEARCHED**

Minimum documentation searched (classification system followed by classification symbols)
Int.Cl. C12N5/071, C07K14/78, C12N1/00, C12N5/074

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | WO 2012/060315 A1 (KUMAMOTO UNIVERSITY) 10 May 2012, examples & US 2014/0199700 A1, examples & EP 2636731 A1 & KR 10-2013-0101557 A & CN 103459591 A | 10<br>1-4<br>5-9 |
| X<br>Y<br>A | OGAKI, S. et al., "Wnt and Notch Signals Guide Embryonic Stem Cell Differentiation into the Intestinal Lineages", Stem Cells, 2013, vol. 31, pp. 1086-1096, abstract, MATERIALS AND METHODS, fig. 1 | 10<br>1-4<br>5-9 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 October 2019 (23.10.2019) | 12 November 2019 (12.11.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/035358

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | WO 2016/147975 A1 (NATIONAL INSTITUTES OF BIOMEDICAL INNOVATION, HEALTH AND NUTRITION) 22 September 2016, claims, examples, fig. 1 & US 2018/0320144 A1, claims, examples, fig. 1 | 10<br>1-4<br>5-9 |
| Y<br>A | JP 2005-525104 A (ACOLOGIX, INC.) 25 August 2005, paragraphs [0001]-[0004] & US 2003/0186891 A1 & WO 2003/066666 A2, pp. 1-2 & EP 1503767 A2 | 1-4<br>5-10 |
| Y<br>A | JP 2009-525030 A (HEALTH RESEARCH INC.) 09 July 2009, claims, paragraphs [0001]-[0008] & US 2007/0207488 A1, claims, paragraphs [0001]-[0007] & WO 2007/092221 A2 & EP 1987164 A2 & CN 101448955 A | 1-4<br>5-10 |
| Y<br>A | JP 2016-530219 A (SALK INSTITUTE FOR BIOLOGICAL STUDIES) 29 September 2016, paragraphs [0034]-[0038] & US 2016/0089385 A1 & WO 2014/197680 A1, pp. 14-15 & EP 3003486 A1 & CN 105451818 A | 1-4<br>5-10 |
| A | WO 2019/021990 A1 (OSAKA UNIVERSITY) 31 January 2019, entire text (Family: none) | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018168957 A
- JP 2019117748 A
- WO 2015125662 A
- WO 2012026531 A
- JP 2012115262 A


### Non-patent literature cited in the description


- **HUBATSCH et al.** *Nat. Protoc.,* 2007, vol. 2, 2111-2119
- **SUN et al.** *Pharm. Res.,* 2002, vol. 19, 4-6
- **TAKAHASHI et al.** *Cell,* 2007, vol. 131, 861-72
- **NAKAMURA et al.** *J. Gastroenterol.,* 2007, vol. 42, 705-10
- **SATO, T. ; CLEVERS, H.** *Science,* 2013, vol. 340, 190-194
- **BUCZACKI et al.** *Nature,* 2013, vol. 495, 65-9
- **CHIBA.** *Stem Cells,* 2006, vol. 24, 2437-47
- **FRE et al.** *Nature,* 2005, vol. 435, 964-8
- **FRE et al.** *Proc. Natl. Acad. Sci. U. S. A.,* 2009, vol. 106, 6309-14
- **GERBE et al.** *J. Cell Biol.,* 2011, vol. 192, 767-80
- **GREGORIEFF et al.** *Nature,* 2015
- **BARKER et al.** *Nature,* 2007, vol. 449, 1003-7
- **SATO et al.** *Nature,* 2009, vol. 459, 262-5
- **SATO et al.** *Gastroenterology,* 2011, vol. 141, 1762-1772
- **SPENCE et al.** *Nature,* 2011, vol. 470, 105-9
- **WATSON et al.** *Nat. Med.,* 2014, 20
- **AMERI et al.** *Stem Cells,* 2010, vol. 28, 45-56
- **HANSSON et al.** *Dev. Biol.,* 2009, vol. 330, 286-304
- **OGAKI et al.** *Stem Cells,* 2013, vol. 31, 1086-96
- **OGAKI et al.** *Sci. Rep.,* 2015, vol. 5, 17297
- **IWAO et al.** *Drug Metab. Dispos.,* 2015, vol. 43, 603-610
- **KODAMA et al.** *Drug Metab. Dispos.,* 2016, 44
- **OZAWA, T. et al.** *Sci. Rep.,* 2015, vol. 5, 16479
- **NEGORO, R. et al.** *Res. Commun.,* 2016, vol. 472, 631-6
- **TAKAHASHI et al.** *Cell,* 2006, vol. 126, 663-676
- **OKITA et al.** *Nature,* 2007, vol. 448, 313-317
- **WERNIG et al.** *Nature,* 2007, vol. 448, 318-324
- **MAHERALI et al.** *Cell Stem Cell,* 2007, vol. 1, 55-70
- **PARK et al.** *Nature,* 2007, vol. 451, 141-146
- **NAKAGAWA et al.** *Nat Biotechnol,* 2008, vol. 26, 101-106
- **WERNIG et al.** *Cell Stem Cell,* 2008, vol. 10, 10-12
- **YU et al.** *Science,* 2007, vol. 318, 1917-1920
- **TAKAHASHI et al.** *Cell,* 2007, vol. 131, 861-872
- **STADTFELD et al.** *Science,* 2008, vol. 322, 945-949
- **SHIRAKI et al.** *Stem Cells,* 2008, vol. 26, 874-85
- **TOSHIAKI TAKEZAWA.** *The Society for Biotechnology Brochure,* 2013, vol. 91, 214-217
- **ESTUDANTE et al.** *Adv. Drug Deliv. Rev.,* 2013, vol. 65, 1340-1356
- **DJUV ; NILSEN.** *Phytother. Res.,* 2008, vol. 22, 1623-1628
- **WRIGHT et al.** *Eur. J. Pharmacol.,* 2011, vol. 672, 70-76
- **COGBURN JN et al.** *Pharm Res.,* 1991, vol. 8, 210-216
- **ARTURSSON.** *J. Pharm. Sci.,* 1990, vol. 79, 476-482
- **OSHIKATA-MIYAZAKI ; TAKEZAWA.** *Cytotechnology,* 2016, vol. 68, 1801-1811
- **YAMAGUCHI et al.** *Toxicol. Sci.,* 2013, vol. 135, 347-355
- **YAMAGUCHI et al.** *J. Appl. Toxicol.,* 2016, vol. 36, 1025-1037
- **AMERI, J. ; STAHLBERG, A. ; PEDERSEN, J. ; JOHANSSON, J. K. ; JOHANNESSON, M. M. ; ARTNER, I. ; SEMB, H.** FGF2 specifies hESC-derived definitive endoderm into foregut/midgut cell lineages in a concentration-dependent manner. *Stem Cells,* 2010, vol. 28, 45-56

- **ARTURSSON, P.** Epithelial Transport Of Drugs In Cell Culture. I: A Model For Studying The Passive Diffusion Of Drugs Over Intestinal Absorbtive (Caco-2) Cells. *J. Pharm. Sci.,* 1990, vol. 79, 476-482
- **ASPLUND, A. ; PRADIP, A. ; VAN GIEZEN, M. ; ASPEGREN, A. ; CHOUKAIR, H. ; REHNSTROM, M. ; JACOBSSON, S. ; GHOSHEH, N. ; EL HAJJAM, D. ; HOLMGREN, S. et al.** One Standardized Differentiation Procedure Robustly Generates Homogenous Hepatocyte Cultures Displaying Metabolic Diversity from a Large Panel of Human Pluripotent Stem Cells. *Stem Cell Rev. Reports,* 2015
- **BARKER, N. ; VAN ES, J. H. ; KUIPERS, J. ; KUJALA, P. ; VAN DEN BORN, M. ; COZIJNSEN, M. ; HAEGEBARTH, A. ; KORVING, J. ; BEGTHEL, H. ; PETERS, P. J. et al.** Identification of stem cells in small intestine and colon by marker gene Lgr5. *Nature,* 2007, vol. 449, 1003-7
- **BUCZACKI, S. J. A ; ZECCHINI, H. I. ; NICHOLSON, A. M. ; RUSSELL, R. ; VERMEULEN, L. ; KEMP, R. ; WINTON, D. J.** Intestinal label-retaining cells are secretory precursors expressing Lgr5. *Nature,* 2013, vol. 495, 65-9
- **CHIBA, S.** Notch signaling in stem cell systems. *Stem Cells,* 2006, vol. 24, 2437-47
- **COGBURN, J. N. ; DONOVAN, M. G. ; SCHASTEEN, C. S.** A model of human small intestinal absorptive cells. 1. Transport barrier. *Pharm. Res.,* 1991, vol. 8, 210-6
- **DJUV, A. ; NILSEN, O. G.** Review of pharmacological effects of Glycyrrhiza radix and its bioactive compounds. *Phytother. Res.,* 2008, vol. 22, 1623-1628
- **ESTUDANTE, M. ; MORAIS, J. G. ; SOVERAL, G. ; BENET, L. Z.** Intestinal drug transporters: An overview. *Adv. Drug Deliv. Rev.,* 2013, vol. 65, 1340-1356
- **FRE, S. ; HUYGHE, M. ; MOURIKIS, P. ; ROBINE, S. ; LOUVARD, D. ; ARTAVANIS-TSAKONAS, S.** Notch signals control the fate of immature progenitor cells in the intestine. *Nature,* 2005, vol. 435, 964-8
- **FRE, S. ; PALLAVI, S. K. ; HUYGHE, M. ; LAE, M. ; JANSSEN, K.-P. ; ROBINE, S. ; ARTAVANIS-TSAKONAS, S. ; LOUVARD, D.** Notch and Wnt signals cooperatively control cell proliferation and tumorigenesis in the intestine. *Proc. Natl. Acad. Sci. U. S. A.,* 2009, vol. 106, 6309-14
- **GERBE, F. ; VAN ES, J. H. ; MAKRINI, L. ; BRULIN, B. ; MELLITZER, G. ; ROBINE, S. ; ROMAGNOLO, B. ; SHROYER, N. F. ; BOURGAUX, J.-F. ; PIGNODEL, C. et al.** Distinct ATOH1 and Neurog3 requirements define tuft cells as a new secretory cell type in the intestinal epithelium. *J. Cell Biol.,* 2011, vol. 192, 767-80
- **GREGORIEFF, A. ; LIU, Y. ; INANLOU, M. R. ; KHOMCHUK, Y. ; WRANA, J. L.** Yap-dependent reprogramming of Lgr5+ stem cells drives intestinal regeneration and cancer. *Nature,* 2015
- **HANSSON, M. ; OLESEN, D. R. ; PETERSLUND, J. M. L. ; ENGBERG, N. ; KAHN, M. ; WINZI, M. ; KLEIN, T. ; MADDOX-HYTTEL, P. ; SERUP, P.** A late requirement for Wnt and FGF signaling during activin-induced formation of foregut endoderm from mouse embryonic stem cells. *Dev. Biol.,* 2009, vol. 330, 286-304
- **HUBATSCH, I. ; RAGNARSSON, E. G. E. ; ARTURSSON, P.** Determination of drug permeability and prediction of drug absorption in Caco-2 monolayers. *Nat. Protoc.,* 2007, vol. 2, 2111-2119
- **IWAO, T. ; KODAMA, N. ; KONDO, Y. ; KABEYA, T. ; NAKAMURA, K. ; HORIKAWA, T. ; NIWA, T. ; KUROSE, K. ; MATSUNAGA, T.** Generation of Enterocyte-Like Cells with Pharmacokinetic Functions from Human Induced Pluripotent Stem Cells Using Small- Molecule Compounds. *Drug Metab. Dispos.,* 2015, vol. 43, 603-610
- **KODAMA, N. ; IWAO, T. ; KATANO, T. ; OHTA, K. ; YUASA, H. ; MATSUNAGA, T.** Characteristic Analysis of Intestinal Transport in Enterocyte-Like Cells Differentiated from Human Induced Pluripotent Stem Cells. *Drug Metab. Dispos.,* 2016, vol. 44, 0-0
- **LANCASTER, M. A. ; KNOBLICH, J. A.** Organogenesis in a dish: modeling development and disease using organoid technologies. *Science,* 2014, vol. 345, 1247125
- **NAKAMURA, T. ; TSUCHIYA, K. ; WATANABE, M.** Crosstalk between Wnt and Notch signaling in intestinal epithelial cell fate decision. *J. Gastroenterol.,* 2007, vol. 42
- **NEGORO, R. ; TAKAYAMA, K. ; NAGAMOTO, Y. ; SAKURAI, F. ; TACHIBANA, M. ; MIZUGUCHI, H.** Modeling of drug-mediated CYP3A4 induction by using human iPS cell-derived enterocyte-like cells. *Biochem. Biophys. Res. Commun.,* 2016, vol. 472, 631-6
- **OGAKI, S. ; SHIRAKI, N. ; KUME, K. ; KUME, S.** Wnt and Notch signals guide embryonic stem cell differentiation into the intestinal lineages. *Stem Cells,* 2013, vol. 31, 1086-96
- **OGAKI, S. ; MOROOKA, M. ; OTERA, K. ; KUME, S.** A cost-effective system for differentiation of intestinal epithelium from human induced pluripotent stem cells. *Sci. Rep.,* 2015, vol. 5, 17297
- **OSHIKATA-MIYAZAKI, A. ; TAKEZAWA, T.** Development of an oxygenation culture method for activating the liver-specific functions of HepG2 cells utilizing a collagen vitrigel membrane chamber. *Cytotechnology,* 2016, vol. 68, 1801-1811
- **SATO, T. ; CLEVERS, H.** Growing Self-Organized Mini-Guts from a Single Intestinal Stem Cell: Mechanisms and Applications. *Science,* 2013, vol. 340, 190-194

- **SATO, T. ; VRIES, R. G. ; SNIPPERT, H. J. ; VAN DE WETERING, M. ; BARKER, N. ; STANGE, D. E. ; VAN ES, J. H. ; ABO, A. ; KUJALA, P. ; PETERS, P. J. et al.** Single Lgr5 stem cells build crypt-villus structures in vitro without a mesenchymal niche. *Nature,* 2009, vol. 459, 262-5
- **SATO, T. ; STANGE, D. E. ; FERRANTE, M. ; VRIES, R. G. J. ; VAN ES, J. H. ; VAN DEN BRINK, S. ; VAN HOUDT, W. J. ; PRONK, A. ; VAN GORP, J. ; SIERSEMA, P. D. et al.** Long-term expansion of epithelial organoids from human colon, adenoma, adenocarcinoma, and Barrett's epithelium. *Gastroenterology,* 2011, vol. 141, 1762-1772
- **SPENCE, J. R. ; MAYHEW, C. N. ; RANKIN, S. A ; KUHAR, M. F. ; VALLANCE, J. E. ; TOLLE, K. ; HOSKINS, E. E. ; KALINICHENKO, V. V ; WELLS, S. I. ; ZORN, A. M. et al.** Directed differentiation of human pluripotent stem cells into intestinal tissue in vitro. *Nature,* 2011, vol. 470, 105-9
- **SUN, D. ; LENNERNAS, H. ; WELAGE, L. S. ; BARNETT, J. L. ; LANDOWSKI, C. P. ; FOSTER, D. ; FLEISHER, D. ; LEE, K. D. ; AMIDON, G. L.** Comparison of human duodenum and Caco-2 gene expression profiles for 12,000 gene sequences tags and correlation with permeability of 26 drugs. *Pharm. Res.,* 2002, vol. 19, 4-6
- **TAKAHASHI, K. ; TANABE, K. ; OHNUKI, M. ; NARITA, M. ; ICHISAKA, T. ; TOMODA, K. ; YAMANAKA, S.** Induction of pluripotent stem cells from adult human fibroblasts by defined factors. *Cell,* 2007, vol. 131, 861-72
- **TAKANO, J. ; MAEDA, K. ; BOLGER, M. B. ; SUGIYAMA, Y.** The prediction of the relative importance of CYP3A/P-glycoprotein to the nonlinear intestinal absorption of drugs by advanced compartmental absorption and transit model. *Drug Metab. Dispos.,* 2016, vol. 44, 1808-1818
- **WATSON, C. L. ; MAHE, M. M. ; MUNERA, J. ; HOWELL, J. C. ; SUNDARAM, N. ; POLING, H. M. ; SCHWEITZER, J. I. ; VALLANCE, J. E. ; MAYHEW, C. N. ; SUN, Y. et al.** An in vivo model of human small intestine using pluripotent stem cells. *Nat. Med.,* 2014, 20
- **WRIGHT, J. A. ; HASLAM, I. S. ; COLEMAN, T. ; SIMMONS, N. L.** Breast cancer resistance protein BCRP (ABCG2)-mediated transepithelial nitrofurantoin secretion and its regulation in human intestinal epithelial (Caco-2) layers. *Eur. J. Pharmacol.,* 2011, vol. 672, 70-76
- **YAMAGUCHI, H. ; KOJIMA, H. ; TAKEZAWA, T.** Vitrigel-eye irritancy test method using HCE-T cells. *Toxicol. Sci.,* 2013, vol. 135, 347-355
- **YAMAGUCHI, H. ; KOJIMA, H. ; TAKEZAWA, T.** Predictive performance of the Vitrigel-eye irritancy test method using 118 chemicals. *J. Appl. Toxicol.,* 2016, vol. 36, 1025-1037